(19) **Europäisches Patentamt European Patent Office Office européen des brevets**

(11) **EP 4 744 631 A1**

(12) **EUROPEAN PATENT APPLICATION**
published in accordance with Art. 153(4) EPC

(43) Date of publication:
**20.05.2026 Bulletin 2026/21**

(21) Application number: **24839788.7**

(22) Date of filing: **10.07.2024**

(51) International Patent Classification (IPC):
**A61F 9/007** (2006.01)

(52) Cooperative Patent Classification (CPC):
**A61F 9/007**

(86) International application number:
**PCT/JP2024/024963**

(87) International publication number:
**WO 2025/013898 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.07.2023 JP 2023113148**

(71) Applicant: **RACTHERA Co., Ltd.**
**Tokyo 103-6012 (JP)**

(72) Inventors:
• **YAMAMOTO, Hiroaki**
  **Kawasaki-shi, Kanagawa 210-8602 (JP)**
• **MIZUNO, Aiko**
  **Tokyo 140-0002 (JP)**
• **MANABE, Koichiro**
  **Tokyo 103-6012 (JP)**
• **KUWAHARA, Atsushi**
  **Kobe-shi, Hyogo 650-0047 (JP)**
• **TANAKA, Kazunari**
  **Suita-shi, Osaka 564-0053 (JP)**

(74) Representative: **TBK**
**Bavariaring 4-6**
**80336 München (DE)**

(54) **CANNULA**

(57) There is provided a cannula having excellent suction property and excellent discharge property. The object is achieved by a cannula including a distal end part that is a bent part bent with respect to a proximal end side part, in which a distal end of the bent part includes a first portion that is located on a bending direction side of the bent part, and a second portion that is located on a side opposite to the bending direction side, the first portion protrudes in an extension direction of the bent part more than the second portion, and has a pointed end that protrudes furthest in a protruding direction of the first portion, and the first portion and the second portion are opposed to each other in parallel.

FIG.2A

**(Cont. next page)**

# FIG.2B

# EP 4 744 631 A1

**Description**

TECHNICAL FIELD

The present invention relates to a cannula.

BACKGROUND ART

Examples of the cannula used by being inserted into a desired portion in a living body include those described in Patent Documents 1 and 2, and Non Patent Document 1.

Citation List

Patent Documents

   Patent Document 1: JP-A-2021-126534
   Patent Document 2: U.S. Patent No. 5,941,250

Non Patent Document

Non Patent Document 1: M. Mandai, et. al, "Autologous Induced Stem-Cell-Derived Retinal Cells for Macular Degeneration", The New England Journal of Medicine, March 16, 2017, 376, pp. 1038-1046

SUMMARY OF INVENTION

Technical Problem

According to the studies by the inventors of the present application, there is room for improvement in suction property and discharge property of a cannula, which is an administration technique of the related art.
The present invention has been made in view of the above-described issues, and an object of the present invention is to provide a cannula having excellent suction property and excellent discharge property.

Solution to Problem

According to the present invention, there is provided a cannula including a distal end part that is a bent part bent with respect to a proximal end side part,

   in which a distal end of the bent part includes a first portion that is located on a bending direction side of the bent part, and a second portion that is located on a side opposite to the bending direction side,
   the first portion protrudes in an extension direction of the bent part more than the second portion, and has a pointed end that protrudes furthest in a protruding direction of the first portion, and
   the first portion and the second portion are opposed to each other in parallel.

In addition, according to the present invention, there is provided a cannula including a distal end part that is a bent part bent with respect to a proximal end side part,

   in which a distal end opening has a flattened shape in which a dimension in a bending direction of the bent part is smaller than a dimension in a direction orthogonal to the bending direction,
   a distal end of the bent part includes a first portion that is located on the bending direction side, and a second portion that is located on a side opposite to the bending direction side,
   the first portion protrudes in an extension direction of the bent part more than the second portion, and has a pointed end that protrudes furthest in a protruding direction of the first portion, and
   the cannula has at least any one of

      an opening area of the distal end opening of 0.20 mm$^2$ or more, or
      a ratio of the opening area of the distal end opening to an outer shape area at a distal end of the cannula, the ratio being 35% or more.

In addition, according to the present invention, there is provided a cannula including a distal end part that is a bent part bent with respect to a proximal end side part,

in which a distal end of the bent part includes a first portion that is located on a bending direction side of the bent part, and a second portion that is located on a side opposite to the bending direction side,
the first portion protrudes in an extension direction of the bent part more than the second portion, and has a pointed end that protrudes furthest in a protruding direction of the first portion,
a wall thickness of the first portion is smaller than a wall thickness of the second portion, and
the first portion and the second portion are opposed to each other in parallel.

In addition, according to the present invention, there is provided a cannula including a distal end part that is a bent part bent with respect to a proximal end side part,

in which a distal end opening has a flattened shape in which a dimension in a bending direction of the bent part is smaller than a dimension in a direction orthogonal to the bending direction,
a distal end of the bent part includes a first portion that is located on the bending direction side, and a second portion that is located on a side opposite to the bending direction side,
the first portion protrudes in an extension direction of the bent part more than the second portion, and has a pointed end that protrudes furthest in a protruding direction of the first portion, and
a wall thickness of the first portion is smaller than a wall thickness of the second portion.

In addition, according to the present invention, there is provided a cannula including a distal end part that is a bent part bent with respect to a proximal end side part,

in which a distal end opening has a flattened shape in which a dimension in a bending direction of the bent part is smaller than a dimension in a direction orthogonal to the bending direction,
a distal end of the bent part includes a first portion that is located on the bending direction side, and a second portion that is located on a side opposite to the bending direction side,
the first portion protrudes in an extension direction of the bent part more than the second portion, and has a pointed end that protrudes furthest in a protruding direction of the first portion, and
the distal end opening has a major axis and a minor axis, and a length of the minor axis is 0.30 mm or more.

In addition, according to the present invention, there is provided a cannula including a distal end part that is a bent part bent with respect to a proximal end side part,

in which a distal end of the bent part includes a first portion that is located on a bending direction side of the bent part, and a second portion that is located on a side opposite to the bending direction side,
the first portion protrudes in an extension direction of the bent part more than the second portion, and has a pointed end that protrudes furthest in a protruding direction of the first portion,
the first portion and the second portion are opposed to each other in parallel, and
the distal end opening has a major axis and a minor axis, and a length of the minor axis is 0.30 mm or more.

Advantageous Effects of Invention

According to the present invention, it is possible to realize a cannula having excellent suction property and excellent discharge property.

BRIEF DESCRIPTION OF DRAWINGS

[FIG. 1] A side view of a cannula according to an embodiment.
[FIG. 2] FIG. 2A is a cut end view taken along the line A-A shown in FIG. 1, and FIG. 2B is a cut end view taken along the line A-A shown in FIG. 2A.
[FIG. 3] A view showing a distal end opening of the cannula according to the present embodiment.
[FIGS. 4] FIGS. 4A and 4B are views showing a bent part of the cannula according to the embodiment and a peripheral structure thereof, wherein FIG. 4A is a cross-sectional view including an axis of the bent part and an axis of a proximal end side part, and FIG. 4B is a side view.

[FIG. 5] FIG. 5A is a cut end view taken along the line B-B shown in FIG. 1, and FIG. 5B is a cut end view taken along the line C-C shown in FIG. 1.

[FIG. 6] A schematic view for describing an example of an operation in a case where cells in the cell recovery container are suctioned by the suction device on which the cannula according to the present embodiment is mounted.

[FIG. 7] FIG. 7 is a schematic view for describing an example of an operation in a case where cells suctioned by the suction device on which the cannula according to the present embodiment is mounted are transplanted into an affected part.

[FIGS. 8] FIGS. 8A and 8B are schematic views for describing an example of an operation in a case where the object suctioned by the suction device on which the cannula according to the present embodiment is mounted is transplanted into the affected part, wherein FIG. 8A shows an operation in a case where a distal end part (bent part) of the cannula is inserted into the biological tissue, and FIG. 8B shows an operation in a case where the object in the cannula is discharged.

[FIG. 9] A captured image of a distal end opening of the cannula according to the present embodiment.

[FIG. 10] A cut end view of a bent part of a cannula according to a modification example of the present embodiment.

[FIG. 11] An image of a retinal sheet (cell sheet) in Example 2, captured with an inverted microscope.

[FIG. 12] An image of the subretinal space of a monkey into which the retinal sheet in Example 4 has been transplanted, captured with a surgical microscope.

[FIG. 13] An image of a luminal surface of a first cannula according to Example 5, captured with a scanning electron microscope.

[FIG. 14] An image of a luminal surface of a second cannula according to Example 5, captured with a scanning electron microscope.

## DESCRIPTION OF EMBODIMENTS

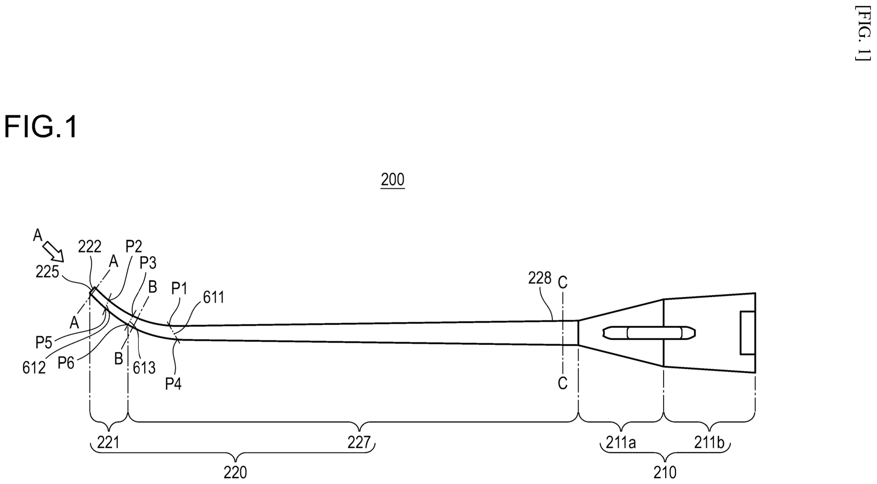

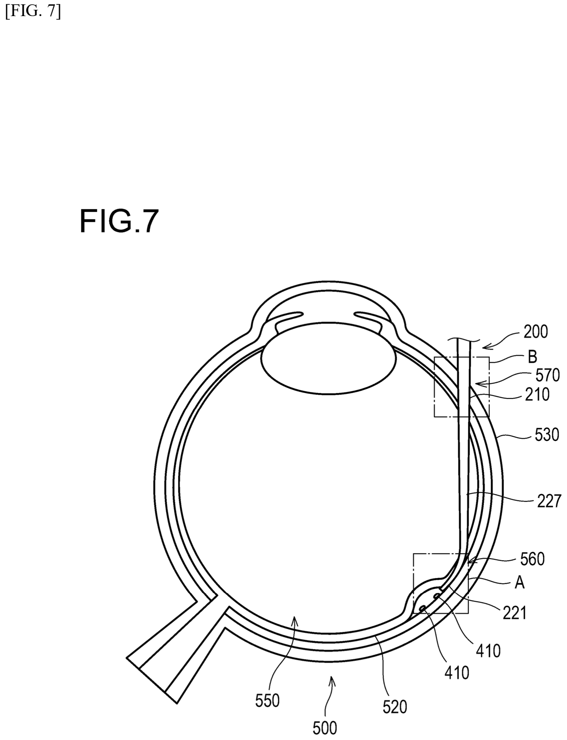

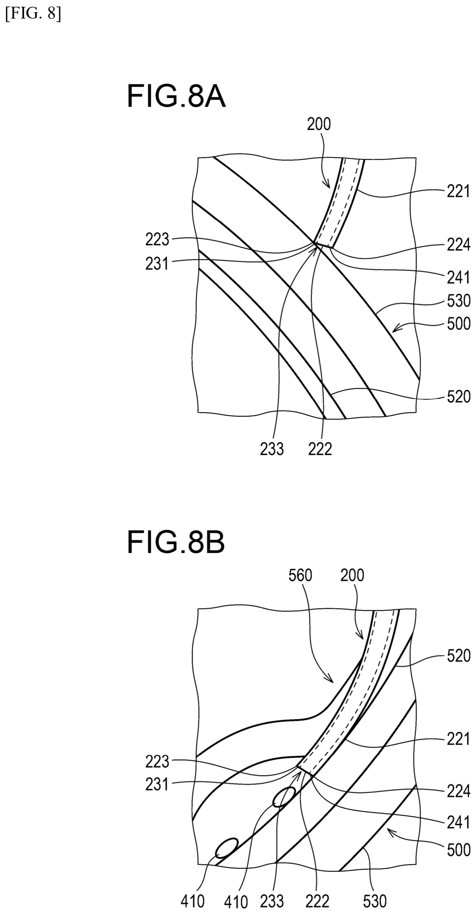

Hereinafter, embodiments of the present invention will be described with reference to FIGS. 1 to 9. It is noted that in all of the drawings, the same components are denoted by the same reference numerals, and the description thereof will be appropriately omitted. In addition, FIG. 8A is a partially enlarged view of a portion corresponding to a portion B of FIG. 7, and FIG. 8B is a partially enlarged view of a portion A shown in FIG. 7.

In addition, various components of the cannula 200 according to the embodiment of the present invention allow that a plurality of components are formed as one member, one component is formed of a plurality of members, one component is a part of another component, a part of one component and a part of another component overlap each other, and the like.

In addition, in the following description, it is assumed that a left direction in FIG. 1 is a distal end side and a right direction is a proximal end side. In addition, the axial direction of the cannula 200 may be simply referred to as an axial direction, and the radial direction of the cannula 200 may be simply referred to as a radial direction. Furthermore, the circumferential direction of the cannula 200 may be simply referred to as a circumferential direction.

As shown in FIGS. 1 to 4B, the distal end part of the cannula 200 according to the present embodiment is a bent part 221 bent with respect to the proximal end side part 227.

The distal end 221a of the bent part 221 includes a first portion 231 located on the bending direction side and a second portion 241 located on a side opposite to the bending direction side.

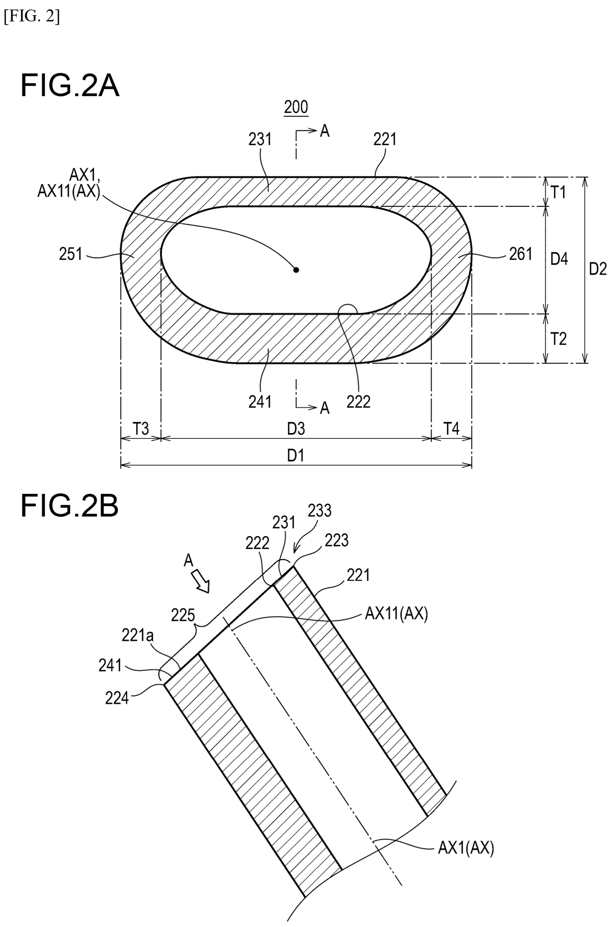

The first portion 231 protrudes in the extension direction (the extending direction of the axis AX11 (see FIG. 2B) of the most distal end part) of the bent part 221 more than the second portion 241 and has a pointed end 233 that protrudes furthest in the protruding direction of the first portion.

That is, the first portion 231 protrudes in the extension direction of the bent part 221 more than the second portion 241. The first portion 231 has a pointed end 233. In addition, the pointed end 233 protrudes in the protruding direction of the first portion 231. The pointed end 233 protrudes furthest in the protruding direction in the first portion 231.

It is noted that in the present invention, the bending direction side is an in-course side (inner curvature side) of bending, and the side opposite to the bending direction side is an out-course side (outer curvature side) of bending.

In addition, in the present invention, the bent part 221 is a portion on the distal end side with respect to a straight line (hereinafter, a boundary line 613 (see FIG. 1)) connecting a point (point P3 shown in FIG. 1) where the curvature on the in-course side of the bending in the cannula 200 is maximum and a point (point P6 shown in FIG. 1) where the curvature on the out-course side of the bending in the cannula 200 is maximum.

In addition, in the present invention, the axis AX of the cannula 200 (axis including the axis AX1 of the bent part 221 and the axis AX11 of the most distal end part of the bent part 221) is the axis of the entire cannula 200 (entire cannula including the pipe wall and the lumen).

In addition, in the present invention, the major axis of the distal end opening 222 is the maximum value of the inner diameter of the distal end opening 222, and the minor axis of the distal end opening 222 is a portion that is a part of a perpendicular bisector line that bisects a major axis (maximum value of the inner diameter) of the inner diameter of the distal end opening 222.

The cannula 200 is used, for example, by being mounted on a suction device (in the present embodiment, a syringe (not shown) which will be described later).

By operating the suction device, it is possible to perform each of a suction operation of suctioning the object (in the present embodiment, the cell sheet 410) into the cannula 200 through the distal end opening 222 and a discharge operation of discharging the object in the cannula 200 from the distal end opening 222.

Then, the discharge operation is performed, for example, in a state in which the distal end part of the cannula 200 is inserted into a desired part of the living body.

It is noted that in the present invention, the suction device on which the cannula 200 is mounted is not particularly limited as long as it is a device capable of performing each of the suction operation and the discharge operation described above.

Here, it is preferable that the first portion 231 and the second portion 241 of the cannula 200 be opposed to each other in parallel.

With such a configuration, the opening width and the opening area of the distal end opening 222 can be sufficiently ensured. More specifically, at least in the region where the first portion 231 and the second portion 241 are disposed in the distal end opening 222, a certain opening width (a separation distance between the inner peripheral surface of the first portion 231 and the inner peripheral surface of the second portion 241 in an opposed direction) and a certain opening area can be ensured. Therefore, for example, compared to a case where the first portion 231 and the second portion 241 are curved or inclined in a direction in which the first portion 231 and the second portion 241 are close to each other, the suction property of the object into the cannula 200 and the discharge property of the object from the cannula 200 are improved. Furthermore, a plurality of objects can be suctioned into the cannula 200 in a single suction operation, and all of the plurality of objects suctioned can be discharged outside the cannula 200 in a single discharge operation. That is, for example, since all of the desired number of cell sheets 410 can be supplied to the affected part (for example, the subretinal space or the vitreous cavity) of the living body in a single suction and discharge operation, the number of times of insertion of the cannula 200 into the living body can be reduced, which can achieve lower invasiveness. It is noted that, here, the expression "a single suction and discharge operation" means suctioning the cell sheet 410 into the cannula 200 in the single suction operation and discharging the absorbed cell sheet 410 to the affected part in the single discharge operation. However, the suction operation of suctioning the cell sheet 410 into the cannula 200 is performed outside the living body (for example, in the cell recovery container 300). Therefore, to realize the low invasive property, it is not always necessary to suction all of the desired number of cell sheets 410 into the cannula 200 in a single suction operation, and it is sufficient that at least all of the plurality of cell sheets 410 that have been suctioned can be discharged in a single discharge operation.

In addition, it is preferable that the first portion 231 and the second portion 241 of the cannula 200 are opposed to each other in parallel, and the wall thickness of the first portion 231 is smaller than the wall thickness of the second portion 241.

With such a configuration, as described above, the opening width and the opening area of the distal end opening 222 can be sufficiently ensured. Therefore, the suction property of the object into the cannula 200 and the discharge property of the object from the cannula 200 are improved. In addition, a plurality of objects (cell sheets 410) can be suctioned into the cannula 200 in a single suction operation, and a plurality of objects (the same as above) can be discharged in a single discharge operation.

Furthermore, as shown in FIG. 8A, since a portion (the pointed end 233 of the first portion 231) that first enters the biological tissue in a case where the cannula 200 is inserted into the affected part of the living body has a thin-walled structure, resistance during insertion can be suppressed, which can achieve lower invasiveness.

On the other hand, since the second portion 241 has a greater wall thickness than the first portion 231, as shown in FIG. 8B, in a case where the object in the cannula 200 is discharged from the distal end opening 222, a separation distance between the distal end opening 222 and the biological tissue around the distal end opening 222 can be ensured. Therefore, immediately after the object is discharged from the distal end opening 222, frictional resistance between the object and the biological tissue around the distal end opening 222 can be suppressed, whereby the object can be easily pushed out to a desired position and supplied (the delivery property is improved). In addition, even in a case where a plurality of objects are supplied, since the frictional resistance between each of the plurality of objects and the biological tissue can be suppressed, good delivery property can be maintained.

In the present invention, the expression "the first portion 231 and the second portion 241 are opposed to each other in parallel" means that the first portion 231 and the second portion 241 are in a relationship of being parallel to each other, as compared with a case where at least the distal end opening 222 has an elliptical shape.

More specifically, in the case of the present embodiment, the expression "the first portion 231 and the second portion 241 are opposed to each other in parallel" means that two conditions of "the first portion 231 and the second portion 241 extend linearly over a range of 2/3 or more of the width dimension (major axis) of the distal end opening 222" and "the variation (displacement) in the separation distance (separation distance in the minor-axis direction) between the first portion 231 and the second portion 241 over the range is sufficiently small" are satisfied.

More specifically, the expression "the first portion 231 extends linearly over a range of 2/3 or more of the major axis of the distal end opening 222" means that the degree of displacement of the first portion 231 is within 14% of the minor axis of the distal end opening 222 over a range of 2/3 or more of the major axis of the distal end opening 222. Preferably, the degree of displacement of the first portion 231 is within 10% of the minor axis of the distal end opening 222 over a range of 2/3 or more of the major axis of the distal end opening 222, and more preferably, the degree of displacement of the first portion 231 is within 7% of the minor axis of the distal end opening 222 over a range of 2/3 or more of the major axis of the distal end opening 222.

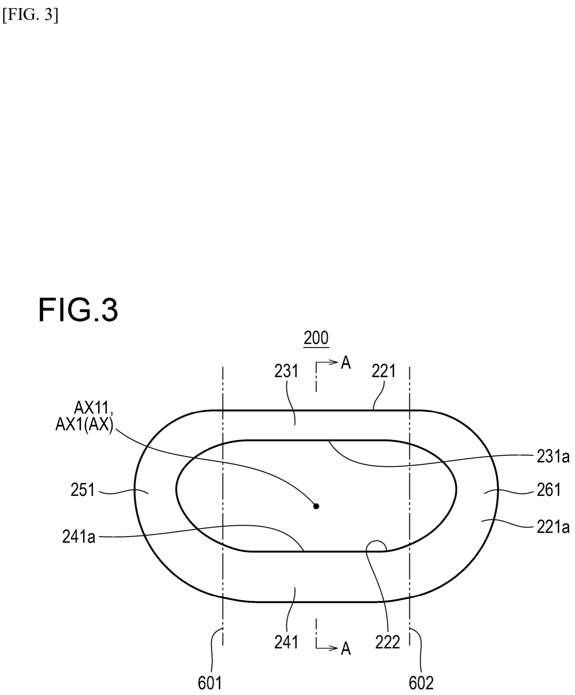

It is noted that the expression "degree of displacement of the first portion 231" as used herein is a ratio of a range (displacement of the inner edge 231a of the first portion 231 in the minor-axis direction) in which the inner edge 231a (FIG. 3) of the first portion 231 is present in the minor-axis direction to the minor axis of the distal end opening 222.

That is, the expression "the first portion 231 extends linearly over a range of 2/3 or more of the major axis of the distal end opening 222" means that a range (displacement of the inner edge 231a of the first portion 231 in the minor-axis direction) in which the inner edge 231a (FIG. 3) of the first portion 231 is present in the minor-axis direction over the range is within 14% of the minor axis of the distal end opening 222.

Similarly, the expression "the second portion 241 extends linearly over a range of 2/3 or more of the major axis of the distal end opening 222" means that the degree of displacement of the second portion 241 is within 14% of the minor axis of the distal end opening 222 over a range of 2/3 or more of the major axis of the distal end opening 222. Preferably, the degree of displacement of the second portion 241 is within 10% of the minor axis of the distal end opening 222 over a range of 2/3 or more of the major axis of the distal end opening 222, and more preferably, the degree of displacement of the second portion 241 is within 7% of the minor axis of the distal end opening 222 over a range of 2/3 or more of the major axis of the distal end opening 222.

It is noted that the expression "degree of displacement of the second portion 241" as used herein is a ratio of a range (displacement of the inner edge 241a of the second portion 241 in the minor-axis direction) in which the inner edge 241a (FIG. 3) of the second portion 241 is present in the minor-axis direction to the minor axis of the distal end opening 222.

That is, the expression "the second portion 241 extends linearly over a range of 2/3 or more of the major axis of the distal end opening 222" means that a range (displacement of the inner edge 241a of the second portion 241 in the minor-axis direction) in which the inner edge 241a (FIG. 3) of the second portion 241 is present in the minor-axis direction over the range is within 14% of the minor axis of the distal end opening 222.

In addition, the expression "the variation (displacement) in the separation distance between the first portion 231 and the second portion 241 over the range is sufficiently small" means that the variation (displacement) in the separation distance (separation distance between the inner edge 231a of the first portion 231 and the inner edge 241a of the second portion 241) between the first portion 231 and the second portion 241 over the range is within 28% of the minor axis of the distal end opening 222. Preferably, the variation (displacement) in the separation distance between the first portion 231 and the second portion 241 over the range is within 20% of the minor axis of the distal end opening 222, and more preferably, the variation (displacement) in the separation distance is within 14% of the minor axis of the distal end opening 222.

In addition, the separation distance (the separation distance between the inner edge 231a of the first portion 231 and the inner edge 241a of the second portion 241) between the first portion 231 and the second portion 241 in the minor-axis direction of the distal end opening 222 over a range of 2/3 or more of the major axis of the distal end opening 222 is 72% or more of the minor axis, preferably 80% or more of the minor axis, more preferably 85% or more of the minor axis, and still more preferably 95% or more of the minor axis.

With such a configuration, a certain opening width (a separation distance between the inner peripheral surface of the first portion 231 and the inner peripheral surface of the second portion 241 in the opposed direction) and thus an opening area of the distal end opening 222 can be better ensured over a range of 2/3 or more of the major axis of the distal end opening 222.

In addition, in the present invention, the distal end 221a of the bent part 221 has a dimension in the axial direction of the bent part 221, and the wall thickness of each of the first portion 231 and the second portion 241 means a wall thickness in the vicinity of the distal end 221a.

In addition, the expression that the wall thickness of the first portion 231 is smaller than the wall thickness of the second portion 241 means that at least the average value of the wall thicknesses of the first portion 231 is smaller than the average value of the wall thicknesses of the second portion 241. Preferably, a ratio (hereinafter, a minimum wall thickness ratio) of a minimum value T1 (T1 shown in FIG. 2A) of a wall thickness of the first portion 231 to a minimum value T2 (T2 shown in FIG. 2A) of a wall thickness of the second portion 241 is 90% or less, more preferably the minimum wall thickness ratio is 80% or less, and still more preferably the minimum wall thickness ratio is 70% or less.

In addition, as shown in FIG. 3, it is preferable that the distal end opening 222 of the cannula 200 according to the present embodiment has a flattened shape in which a dimension in a bending direction of the bent part 221 is smaller than a dimension in a direction orthogonal to the bending direction. Furthermore, it is preferable that the cannula 200 has at least one of an opening area (inner circle area) of the distal end opening 222 of 0.20 mm² or more, or a ratio (opening area ratio) of the opening area of the distal end opening to an outer shape area (outer circle area) at the distal end (see FIG. 3) of the cannula, the ratio being 35% or more.

It is noted that the expression "dimension in a bending direction of the bent part 221 is smaller than a dimension in a direction orthogonal to the bending direction" means a dimensional relationship in a case where the bent part 221 is directly opposed to the distal end opening 222 (in a case where the bent part 221 is viewed from the distal end side in the extending direction of the axis AX11 of the most distal end part of the bent part 221 (more specifically, in a case where the bent part 221 is viewed in the direction of the arrow A shown in FIGS. 1 and 2B)). In addition, FIG. 3 is a view of the bent part 221 as viewed from the distal end side in the extending direction of an axis AX11 of the most distal end part of the bent part 221.

In addition, examples of the "flattened shape" as used herein include a rounded rectangular shape and shapes among super-ellipses for which n > 2.

Here, the super-ellipse is a closed curve represented by the following equation (1).

$$|x/a|^n + |x/b|^n = 1 \ ... \ (1)$$

Here, n, a, and b are each a positive number, and $\|$ indicates an absolute value.

Since the cannula 200 has the opening area and the opening area ratio of the distal end opening 222, the suction property and the discharge property of the cannula 200 can be sufficiently ensured.

Furthermore, since the distal end opening 222 of the cannula 200 has a flattened shape, in the distal end opening 222, the dimension (in the present embodiment, the major axis of the distal end opening 222) in the direction orthogonal to the bending direction can be set to be larger while ensuring the dimension (in the present embodiment, the minor axis of the distal end opening 222) of the bent part 221 in the bending direction to be equal to or greater than a certain value over a certain range. Therefore, the suction property of the object into the cannula 200 and the discharge property of the object from the cannula 200 are improved. In addition, a plurality of objects (cell sheets 410) can be suctioned into the cannula 200 in a single suction operation, and a plurality of objects (the same as above) can be discharged in a single discharge operation.

In addition, as shown in FIG. 3, it is also preferable that the distal end opening 222 of the cannula 200 has a flattened shape in which a dimension of the bent part 221 in a bending direction is smaller than a dimension of the bent part 221 in a direction orthogonal to the bending direction, and a wall thickness of the first portion 231 is smaller than a wall thickness of the second portion 241.

Even with such a configuration, since the distal end opening 222 of the cannula 200 has a flattened shape, the opening width and the opening area of the distal end opening 222 can be sufficiently ensured. More specifically, in the distal end opening 222, the dimension (in the present embodiment, the major axis of the distal end opening 222) in the direction orthogonal to the bending direction can be set to be larger while ensuring the dimension (in the present embodiment, the minor axis of the distal end opening 222) of the bent part 221 in the bending direction to be equal to or greater than a certain value over a certain range. Therefore, the suction property of the object into the cannula 200 and the discharge property of the object from the cannula 200 are improved. In addition, a plurality of objects (cell sheets 410) can be suctioned into the cannula 200 in a single suction operation, and a plurality of objects (the same as above) can be discharged in a single discharge operation.

Furthermore, as described above, as shown in FIG. 8A, since a portion (the pointed end 233 of the first portion 231) that first enters the biological tissue in a case where the cannula 200 is inserted into the affected part of the living body has a thin-walled structure, resistance during insertion can be suppressed, which can achieve lower invasiveness.

On the other hand, since the second portion 241 has a greater wall thickness than the first portion 231, as shown in FIG. 8B, in a case where the object in the cannula 200 is discharged from the distal end opening 222, a separation distance between the distal end opening 222 and the biological tissue around the distal end opening 222 can be ensured. Therefore, immediately after the object is discharged from the distal end opening 222, frictional resistance between the object and the biological tissue around the distal end opening 222 can be suppressed, whereby the object can be easily pushed out to a desired position and supplied (the delivery property is improved). In addition, even in a case where a plurality of objects are supplied to a desired position, since the frictional resistance between each of the plurality of objects and the biological tissue can be suppressed, good delivery property can be maintained.

It is noted that from the viewpoint of insertion property into an affected part of a living body (for example, a subretinal space or a vitreous cavity) and the viewpoint of the shape retention of the first portion 231 and the second portion 241, it is preferable that the distal end part of the cannula 200 has an appropriate wall thickness.

Therefore, the opening area (inner circle area) of the distal end opening 222 is preferably 0.20 mm² or more and more preferably 0.25 mm$^2$ or more within a range in which an appropriate wall thickness of the distal end part of the cannula 200 can be ensured. In addition, the opening area of the distal end opening 222 is, for example, 50 mm$^2$ or less, more preferably 20 mm$^2$ or less, still more preferably 10 mm$^2$ or less, still more preferably 5.0 mm$^2$ or less, still more preferably 2.0 mm$^2$ or less, still more preferably 1.0 mm$^2$ or less, still more preferably 0.80 mm$^2$ or less, still more preferably 0.50 mm$^2$ or less, and most preferably 0.30 mm$^2$ or less.

Similarly, at the distal end of the cannula 200, the ratio (opening area ratio) of the opening area of the distal end opening 222 to the outer shape area (outer circle area) is preferably 35% or more and more preferably 40% or more within a range in which it is possible to ensure an appropriate wall thickness of the distal end part of the cannula 200. In addition, the opening area ratio of the distal end opening 222 is, for example, 70% or less, more preferably 60% or less, and most preferably 50% or less.

With such a configuration, it is possible to improve the suction property and the discharge property of the cannula 200 while ensuring the insertion property of the cannula 200.

In the cannula 200 having the opening area and the opening area ratio of the distal end opening 222, the first portion 231 and the second portion 241 are opposed to each other in parallel, whereby the suction property and the discharge property of the cannula 200 can be further improved.

Similarly, in the cannula 200 having the opening area and the opening area ratio of the distal end opening 222, the distal end opening 222 has a flattened shape and the wall thickness of the first portion 231 is smaller than the wall thickness of the second portion 241, whereby the suction property and the discharge property of the cannula 200 can be further improved.

Here, the cannula 200 according to the present embodiment includes, as an example, all of the four features of the first feature that "the first portion 231 and the second portion 241 of the cannula 200 are opposed to each other in parallel" described above, the second feature that "the distal end opening 222 has a flattened shape, and the cannula 200 has at least any one of the opening area of the distal end opening 222 of 0.20 mm$^2$ or more or the opening area ratio of the distal end opening 222 of 35% or more" described above, the third feature that "the wall thickness of the first portion 231 is smaller than the wall thickness of the second portion 241 and the first portion 231 and the second portion 241 of the cannula 200 are opposed to each other in parallel" described above, and the fourth feature that "the distal end opening 222 has a flattened shape and the wall thickness of the first portion 231 is smaller than the wall thickness of the second portion 241" described above.

That is, the distal end opening 222 of the cannula 200 has a flattened shape, the wall thickness of the first portion 231 is smaller than the wall thickness of the second portion 241, and the first portion 231 and the second portion 241 are opposed to each other in parallel. In addition, in the case of the present embodiment, the cannula 200 has an opening area of the distal end opening 222 of 0.20 mm$^2$ or more and an opening area ratio of the distal end opening 222 of 35% or more.

With such a configuration, it is possible to realize the cannula 200 having good suction property, good discharge property, good insertion property, and good supply property of the object as described above.

However, the present invention is not limited to this example, and the cannula 200 may not necessarily include all of the four features of the first feature, the second feature, the third feature, and the fourth feature described above.

More specifically, as a modification example, the cannula 200 may include the second feature among the four features, but may not include the first feature, the third feature, and the fourth feature.

Even in such a case, the suction property and the discharge property of the cannula 200 can be sufficiently ensured by the second feature as described above.

In addition, as another modification example, the cannula 200 may include the first feature and the second feature among the four features, but may not include the third feature and the fourth feature.

Even in such a case, the suction property and the discharge property of the cannula 200 can be sufficiently ensured by the first feature and the second feature as described above.

However, it is more preferable that the cannula 200 has all of the above four features.

With such a configuration, the suction property and the discharge property of the cannula 200 can be better ensured.

In a case where the cannula 200 does not include the third feature or the fourth feature among the four features, the wall thickness of the first portion 231 may be, for example, larger than the wall thickness of the second portion 241 or may be equal to the wall thickness of the second portion 241.

In addition, in a case where the cannula 200 does not include the first feature or the third feature among the four features, as an example, the first portion 231 may be curved convexly toward one side in the major-axis direction of the bent part 221, and the second portion 241 may be curved convexly toward the other side in the major-axis direction of the bent part 221. In this case, it is preferable that the curvature radius of the inner peripheral surface of the first portion 231 is larger than the curvature radius of the inner peripheral surface of the second portion 241. With such a configuration, it is possible to suppress the first portion 231 from interfering with the cell sheet 410 (particularly, a portion thereof that is convex in a substantially dome shape). In addition, the curvature radius of the inner peripheral surface of the second portion 241 is small, whereby the storage solution 420 (or the administration solution) can be guided to the second portion 241 and smoothly flow at a more stable flow rate.

In addition, in the cannula 200 having a flattened shape in which a dimension of the bent part 221 in the bending direction is smaller than a dimension in a direction orthogonal to the bending direction, it is preferable that the distal end opening 222 has a major axis and a minor axis, and a length of the minor axis is 0.30 mm or more. More preferably, the length of the minor axis of the distal end opening 222 is 0.35 mm or more. In addition, the length of the minor axis of the distal end opening 222 is, for example, 5.0 mm or less, more preferably 3.0 mm or less, more preferably 1.5 mm or less, more preferably 0.9 mm or less, more preferably 0.6 mm or less, more preferably 0.5 mm or less, and most preferably 0.4 mm or less.

With such a configuration, the suction property of the object into the cannula 200 and the discharge property of the object from the cannula 200 are improved. More specifically, for example, even in a case where the object is the cell sheet 410 and a part of the cell sheet 410 is convex in a substantially dome shape, the cell sheet 410 can be smoothly suctioned and discharged through the distal end opening 222.

In addition, in the cannula 200 in which the first portion 231 and the second portion 241 are opposed to each other in parallel, it is preferable that the distal end opening 222 has a major axis and a minor axis and the length of the minor axis is 0.30 mm or more. More preferably, the length of the minor axis of the distal end opening 222 is 0.35 mm or more. In addition, the length of the minor axis of the distal end opening 222 is, for example, 5.0 mm or less, more preferably 3.0 mm or less, more preferably 1.5 mm or less, more preferably 0.9 mm or less, more preferably 0.6 mm or less, more preferably 0.5 mm or less, and most preferably 0.4 mm or less.

Even with such a configuration, the suction property of the object into the cannula 200 and the discharge property of the object from the cannula 200 are improved. More specifically, for example, even in a case where the object is the cell sheet 410 and a part of the cell sheet 410 is convex in a substantially dome shape, the cell sheet 410 can be smoothly suctioned and discharged through the distal end opening 222.

Here, cell transplantation therapy is desired as a new treatment method for retinal degenerative diseases including age-related macular degeneration and retinitis pigmentosa. However, the above-described cannula of the related art has room for improvement from the viewpoint of easy insertion into the eye.

In contrast, with the cannula 200 according to the present embodiment, since the wall thickness of the first portion 231 is smaller than the wall thickness of the second portion 241 as described above, the resistance in a case where the cannula 200 is inserted into the affected part of the living body can be suppressed. Therefore, the cannula 200 can be easily inserted into the eye.

In addition, in the case of the transplantation of the cell suspension, since the size of the cell is small, a well-known administration needle can be used. On the other hand, in the case of the transplantation of the cell aggregates and the biological tissue, since the size of the cell aggregates and the biological tissue is large, it has been difficult to administer the cell aggregates and the biological tissue into the eye using a well-known administration needle.

In contrast, with the cannula 200 according to the present embodiment, since the opening width and the opening area of the distal end opening 222 can be well ensured as described above, even a cell aggregate or biological tissue having a large size can be satisfactorily suctioned and discharged (transplanted) into the eye.

In addition, according to the studies by the inventors of the present application, for the treatment of age-related macular degeneration, transplantation of cells, cell aggregates, and biological tissues to the central portion of the retina (around the macular region) is expected. However, the above-described cannula of the related art has room for improvement from the viewpoint of stably transplanting cells, cell aggregates, and biological tissues to the central portion of the retina.

In contrast, with the cannula 200 according to the present embodiment, since good suction property and good discharge property can be realized as described above, it is possible to stably transplant cells, cell aggregates, and biological tissues into the center portion of the retina.

In addition, according to the studies by the inventors of the present application, it is considered that in retinitis pigmentosa, since a wide range of the retina of the subject is degenerated, it is preferable to transplant cells, cell aggregates, and biological tissues in a relatively wide area in the eye. However, it has been difficult to transplant cells into a wide area with a well-known administration needle.

In contrast, with the cannula 200 according to the present embodiment, since the opening width and the opening area of the distal end opening 222 can be sufficiently ensured as described above, it is easy to transplant cells, cell aggregates, and tissues into a wide area in the eye.

Hereinafter, the cannula 200 according to the present embodiment will be described in more detail.

As shown in FIG. 1, the cannula 200 is, for example, a tubular member that is tapered toward a distal end side. As described above, the distal end part of the cannula 200 is the bent part 221 bent with respect to the proximal end side part 227.

More specifically, the cannula 200 includes a mounting part 210 that is a part to be mounted on a distal end part (not shown) of the suction device, and a conduit part 220 that is a part on the distal end side with respect to the mounting part 210 and through which the object (cell sheet 410) is guided to pass.

During the suction operation, the object (cell sheet 410) is suctioned into the conduit part 220 of the cannula 200, and during the discharge operation, the object in the conduit part 220 of the cannula 200 passes through the conduit part 220 and is discharged from the distal end opening 222.

In the case of the present embodiment, in the conduit part 220, a portion on the distal end side with respect to the above-described boundary line 613 is the bent part 221, and a portion on the proximal end side with respect to the boundary line 613 is the proximal end side part 227.

Here, as shown in FIG. 1, in the case of the present embodiment, a portion from the proximal end side part 227 to the distal end of the bent part 221 is bent in an arc shape.

Here, the expression "a portion from the proximal end side part 227 to the distal end of the bent part 221 is bent in an arc shape" means that at least a portion from the portion on the proximal end side with respect to the boundary line 613 to the portion on the distal end side with respect to the boundary line 613 is bent in an arc shape. In other words, it means that in the cannula 200, the portion bent in an arc shape is present across at least the boundary line 613.

With such a configuration, for example, it is possible to realize smooth flow property of the object from the proximal end side part 227 toward the bent part 221, as compared with a case where the bent part 221 is bent in an angular shape (broken-line shape) with respect to the proximal end side part 227.

However, the present invention is not limited to this example, and for example, a portion from the proximal end side part 227 to the distal end of the bent part 221 may be bent in an angular shape (broken-line shape).

More specifically, in the case of the present embodiment, the proximal end side part 227 includes a bending start point P1 on the in-course side and a bending start point P4 on the out-course side (see FIG. 1). In the proximal end side part 227, a portion on the distal end side with respect to a straight line (hereinafter, a boundary line 611 (see FIG. 1)) connecting the start point P1 and the start point P4 is slightly bent in an arc shape. On the other hand, in the proximal end side part 227, a portion on the proximal end side with respect to the boundary line 611 extends linearly. However, in the present invention, the entire proximal end side part 227 may be formed in, for example, a linear shape or an arc shape.

In addition, in the present embodiment, the bent part 221 includes a bending end point P2 on the in-course side and a bending end point P5 on the out-course side (see FIG. 1). In the bent part 221, a portion on the distal end side with respect to a straight line (hereinafter, a boundary line 612 (see FIG. 1)) connecting the end point P2 and the end point P5 extends linearly. On the other hand, in the bent part 221, a portion on the proximal end side with respect to the boundary line 612 is bent in an arc shape. However, the present invention is not limited to this example, and the entire bent part 221 may be formed in, for example, an arc shape or a linear shape.

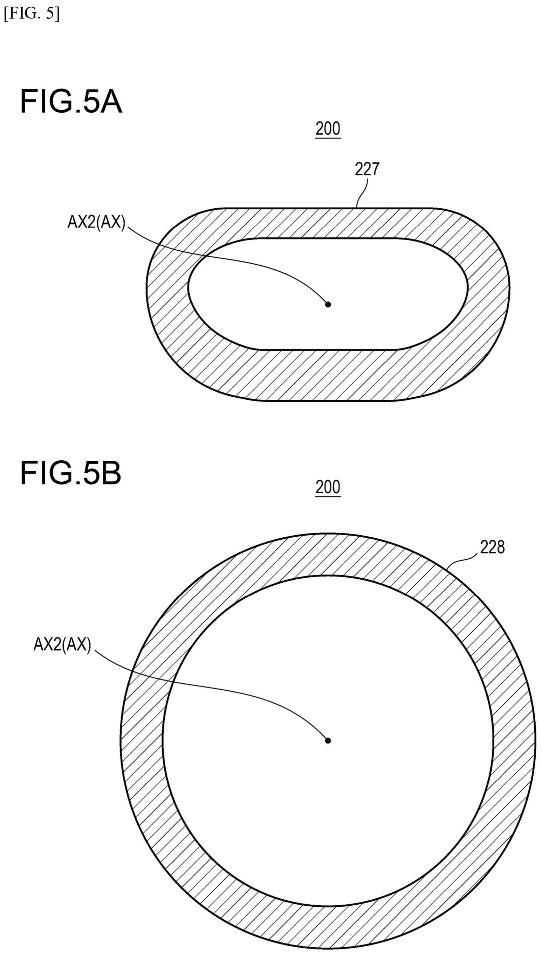

In addition, as an example, the lumen cross-sectional shape of the proximal end part 228 in the proximal end side part 227 is a true circular shape (see FIG. 5B), and the lumen cross-sectional shape of a portion from the intermediate portion of the proximal end side part 227 to the distal end opening 222 is a flattened shape (see FIG. 5A).

Here, the expression "the lumen cross-sectional shape of the proximal end part 228 is a true circular shape" means that the roundness of the lumen cross section of the proximal end part 228 is at least 1/20 or less of the maximum value A of the diameter of the lumen cross section, and preferably 1/25 or less of the maximum value A. It is noted that the roundness is obtained in accordance with JIS-B-0621. More specifically, in a case where a lumen cross section (circular shape) is sandwiched between two concentric geometric circles, a difference in radius between the two geometric circles in a case where the interval between the two geometric circles is minimized is the roundness. In the present embodiment, the roundness of the lumen cross section of the proximal end part 228 is a value obtained by measuring the outer diameter of the lumen cross section of the proximal end part 228 at two points while dividing the outer diameter into 4 to 8 equal parts, and dividing the difference between the maximum value A and the minimum value B of the measured values (the diameter of the lumen cross section) by 2. It is noted that as an example, the measurement of the roundness is performed using a micrometer.

With such a configuration, the lumen cross-sectional shape is a flattened shape from a portion on the proximal end side with respect to the bent part 221. Therefore, in a case where the aspect ratio (vertical-to-horizontal ratio) of the object is different, it is possible to pass the object through the bent part 221 after orienting the object to conform to the flattened shape (the direction of the object to be discharged is changed). Therefore, as compared with a case where the object is oriented while passing through the bent part 221 from the proximal end side part 227, the object can smoothly pass through the bent part 221 from the proximal end side part 227 without being caught or clogged in the intermediate portion.

However, the present invention is not limited to this example, and the lumen cross-sectional shape of the portion from the intermediate portion of the proximal end side part 227 to the distal end opening 222 may be, for example, a true circular shape.

In addition, in the present invention, the lumen cross-sectional shape of the proximal end part 228 is not limited to a true circular shape, and can be appropriately set according to, for example, the shape of the distal end part of the suction device on which the cannula 200 is mounted.

In the present embodiment, the diameter of the mounting part 210 of the cannula 200 is reduced toward the distal end side. More specifically, the taper angle changes in the intermediate portion of the mounting part 210 in the axial direction.

More specifically, the mounting part 210 includes, for example, a large-diameter part 211b that is a most proximal end part of the cannula 200, and a small-diameter part 211a that is connected to a distal end of the large-diameter part 211b.

Each of the inner diameter and the outer diameter of the large-diameter part 211b is gradually reduced toward the distal end side. Each of the inner diameter and the outer diameter of the small-diameter part 211a is gradually reduced toward the distal end side by an amount of change that is larger than an amount of change of each of the inner diameter and the outer diameter of the large-diameter part 211b due to the reduction in diameter.

In addition, as shown in FIG. 3, the distal end 221a of the bent part 221 includes, for example, a third portion 251 that connects one end of the first portion 231 and one end of the second portion 241 in the circumferential direction, and a fourth portion 261 that connects the other end of the first portion 231 and the other end of the second portion 241 in the circumferential direction.

In addition, the wall thickness of the third portion 251 and the wall thickness of the fourth portion 261 are larger than the wall thickness of the first portion 231.

Accordingly, since the wall thickness of the first portion 231 is smaller than the wall thickness of the second portion 241, and the wall thickness of each of the third portion 251 and the fourth portion 261 can be sufficiently ensured, good shape retention of the distal end opening 222 can be realized.

However, the present invention is not limited to this example, and the wall thickness of the third portion 251 and the wall thickness of the fourth portion 261 may be, for example, substantially equal to the wall thickness of the first portion 231 or may be smaller than the wall thickness of the first portion 231.

Here, in FIG. 3, a range of 2/3 of the width dimension (major axis) of the distal end opening 222 is indicated by an imaginary line 601 and an imaginary line 602. The imaginary line 601 is a straight line extending along the minor-axis direction of the distal end opening 222 and is disposed at a position offset by 1/3 of the major axis from the center of the distal end opening 222 to one side in the major-axis direction. Similarly, the imaginary line 602 is a straight line extending along the minor-axis direction of the distal end opening 222, and is disposed at a position offset by 1/3 of the major axis from the center of the distal end opening 222 to the other side in the major-axis direction. The range of 2/3 of the width dimension (major axis) of the distal end opening 222 is a range between the imaginary line 601 and the imaginary line 602. In the case of the present embodiment, as an example, in the distal end 221a of the bent part 221, a portion between the imaginary line 601 and the imaginary line 602 among the portions located on the bending direction side is the first portion 231. In addition, in the distal end 221a of the bent part 221, a portion between the imaginary line 601 and the imaginary line 602 among the portions located on the side opposite to the bending direction side is the second portion 241. That is, as an example, the entire first portion 231 and the entire second portion 241 are opposed to each other in parallel.

More specifically, as shown in FIG. 3, in the case of the present embodiment, at the distal end 221a of the bent part 221, a portion on one side of the bent part 221 in the major-axis direction is the third portion 251 with reference to the imaginary line 601. In addition, in the distal end 221a of the bent part 221, a portion on the other side of the bent part 221 in the major-axis direction is the fourth portion 261 with reference to the imaginary line 602.

In addition, the wall thickness of the third portion 251 gradually and continuously increases from one end of the first portion 231 toward one end of the second portion 241 in the circumferential direction. Similarly, the wall thickness of the fourth portion 261 gradually and continuously increases from the other end of the first portion 231 toward the other end of the second portion 241 in the circumferential direction.

As shown in FIG. 2A, the lumen cross-sectional shape of the bent part 221 is a substantially elongated circular shape that is elongated in one direction, and the substantially elongated circular shape includes portions (a first portion 231 and a second portion 241) that extend in parallel with each other and semi-arc-shaped portions (a third portion 251 and a fourth portion 261). That is, in the lumen cross section of the bent part 221, the portions (the first portion 231 and the second portion 241) extending in parallel with each other and the semi-arc-shaped portions (the third portion 251 and the fourth portion 261) are disposed to be continuous with each other.

More specifically, in a lumen cross section of the bent part 221 (see FIG. 2A), the first portion 231 and the second portion 241 are formed substantially flat. In addition, the first portion 231 and the second portion 241 are opposed to each other in parallel in, for example, the minor-axis direction of the bent part 221.

On the other hand, in the lumen cross section of the bent part 221, the third portion 251 is formed in a substantially arc shape that is convex toward one side in the major-axis direction of the bent part 221, and the fourth portion 261 is formed in a substantially arc shape that is convex toward the other side in the major-axis direction of the bent part 221.

With such a configuration, a certain opening width (a separation distance between the inner peripheral surface of the first portion 231 and the inner peripheral surface of the second portion 241 in the opposed direction) and an opening area can be sufficiently ensured over a certain range in the major-axis direction of the bent part 221, and the major axis of the bent part 221 and thus the distal end opening 222 can be set to be larger. Therefore, even in a case where a part of the cell sheet 410 is convex in a substantially dome shape as described above, the cell sheet 410 can be smoothly suctioned and discharged through the distal end opening 222.

In the present embodiment, the average value of the wall thicknesses of the first portion 231 is smaller than each of the average value of the wall thicknesses of the third portion 251 and the average value of the wall thicknesses of the fourth portion 261.

The average value of the wall thicknesses of the second portion 241 is larger than each of the average value of the wall thicknesses of the third portion 251 and the average value of the wall thicknesses of the fourth portion 261.

In addition, it is preferable that each of a minimum value of the wall thickness of the third portion 251 and a minimum value of the wall thickness of the fourth portion 261 is larger than the minimum value of the wall thickness of the first portion 231.

It is preferable that each of a minimum value of the wall thickness of the third portion 251 and a minimum value of the wall thickness of the fourth portion 261 is smaller than the minimum value of the wall thickness of the second portion 241.

With such a configuration, the major axis of the bent part 221 and thus the distal end opening 222 can be set to be even larger while better ensuring a certain opening width (a separation distance between the inner peripheral surface of the first portion 231 and the inner peripheral surface of the second portion 241 in the opposed direction) and an opening area over a certain range in the major-axis direction of the bent part 221.

In the present embodiment, for example, the major axis (D1 shown in FIG. 2A) of the bent part 221 is preferably 0.5 mm or more and 1.5 mm or less and more preferably 0.9 mm or more and 1.3 mm or less.

The minor axis (D2 shown in FIG. 2A) of the bent part 221 is, for example, preferably 0.3 mm or more and 1.0 mm or less and more preferably 0.5 mm or more and 0.8 mm or less.

The major axis (D3 shown in FIG. 2A) of the distal end opening 222 is, for example, preferably 0.3 mm or more and 1.2 mm or less and more preferably 0.7 mm or more and 1.0 mm or less.

The minor axis (D4 shown in FIG. 2A) of the distal end opening 222 is, for example, preferably 0.2 mm or more and 0.9 mm or less and more preferably 0.3 mm or more and 0.6 mm or less.

The minimum value (T1 shown in FIG. 2A) of the wall thickness of the first portion 231 is, for example, preferably 0.05 mm or more and 0.15 mm or less and more preferably 0.07 mm or more and 0.12 mm or less.

The minimum value (T2 shown in FIG. 2A) of the wall thickness of the second portion 241 is, for example, preferably 0.1 mm or more and 0.2 mm or less and more preferably 0.12 mm or more and 0.17 mm or less.

The thickness dimension (T3 shown in FIG. 2A) of an intermediate portion (intermediate in the minor-axis direction) of the third portion 251 is, for example, preferably 0.1 mm or more and 0.2 mm or less and more preferably 0.13 mm or more and 0.19 mm or less.

A thickness dimension (T4 shown in FIG. 2A) of an intermediate portion (intermediate in the minor-axis direction) of the fourth portion 261 is, for example, preferably 0.1 mm or more and 0.2 mm or less and more preferably 0.13 mm or more and 0.19 mm or less.

In addition, the distal end 221a of the bent part 221 includes, for example, an inclined part 225 inclined with respect to the axis AX1 of the bent part 221. In other words, the first portion 231 includes, for example, the inclined part 225 inclined with respect to the axis AX1 of the bent part 221.

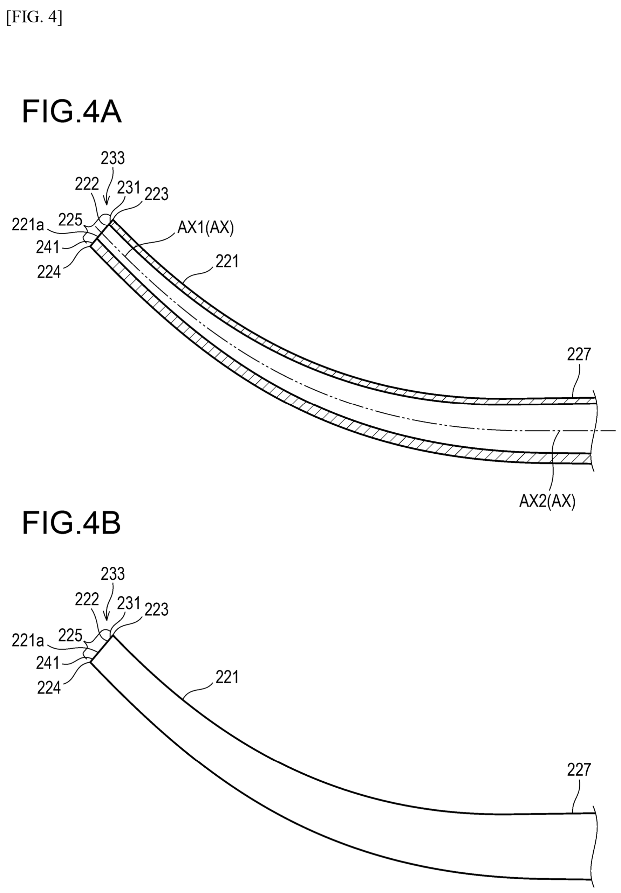

In a cross section (see FIGS. 2B and 4A) including the axis AX1 of the bent part 221 and the axis of the proximal end side part 227, the inclined part 225 extends from the corner 223 on the bending direction side at the distal end 221a of the bent part 221 toward a side opposite to the bending direction side.

The corner 223 on the bending direction side as used herein means a corner of a boundary between an outer shape line of the pipe wall of the bent part 221 on the bending direction side and the distal end 221a of the bent part 221 in a cross section including the axis AX1 of the bent part 221 and the axis of the proximal end side part 227.

With such a configuration, since the bent part 221 can smoothly enter the biological tissues in a case where the cannula 200 is inserted into the affected part of the living body, resistance during insertion can be suppressed, which can achieve lower invasiveness.

Furthermore, in the case of the present embodiment, in a cross section (see FIGS. 2B and 4A) including the axis AX1 of the bent part 221 and the axis AX2 of the proximal end side part 227, an angle of the corner 223 at the distal end 221a of the bent part 221 on the bending direction side is acute (bevel angle). In other words, in a cross section including the axis AX1 of the bent part 221 and the axis AX2 of the proximal end side part 227, an angle of the corner 223 on the bending direction side in the first portion 231 is acute. More specifically, the angle of the corner 223 on the bending direction side in the first portion 231 is at least 50° or more and 85° or less and preferably 60° or more and 85° or less. In the present embodiment, the angle of the corner 223 on the bending direction side in the first portion 231 is, for example, about 75° (75° ± 5°, 75° ± 3°, 75° ± 2°, 75° ± 1°) or about 70° (70° ± 5°, 70° ± 3°, 70° ± 2°, 70° ± 1°). With such a configuration, for example, in a case of a procedure of transplanting a cell sheet 410, which will be described later, into subretinal space in the eyeball 500, it is easy to perform an operation of inserting the distal end part of the cannula 200 into the subretinal space in the eyeball of the subject and thereby lifting the retina, and the cell sheet 410 can be smoothly discharged from the distal end opening 222.

It is noted that in the case of the present embodiment, the pointed end 233 is a corner 223 on the bending direction side at the distal end 221a of the bent part 221. However, in the present invention, a portion other than the corner 223 on the bending direction side may be the pointed end 233.

More specifically, in a cross section including the axis AX1 of the bent part 221 and the axis of the proximal end side part 227, the inclined part 225 extends from the corner 223 on the bending direction side at the distal end 221a of the bent part 221 toward the corner 224 on a side opposite to the bending direction side.

As described above, an angle of the corner 223 at the distal end 221a of the bent part 221 on the bending direction side is acute.

On the other hand, an angle of the corner 224 at the distal end 221a of the bent part 221 on a side opposite to the bending direction side is obtuse. As shown in FIG. 8B, a corner 224 at the distal end of the bent part 221 on a side opposite to the bending direction side is a portion that is likely to come into contact with a biological tissue (for example, a retina 520 which will be described later) in a case where the object is discharged from the distal end opening 222, and since an angle of the portion is obtuse, a configuration with lower invasiveness can be realized in the cannula 200.

It is noted that as in a modification example described later, the corner 224 on the side opposite to the bending direction side may have an R-chamfered shape.

In addition, on the luminal surface of the cannula 200, the number of fissures having a width of 10 $\mu$m or more is preferably less than 10 per 0.045 mm$^2$.

With such a configuration, since the luminal surface of the cannula 200 is smoother, frictional resistance between the object and the luminal surface of the cannula 200 can be suppressed. Therefore, the suction property of the object into the cannula 200 and the discharge property of the object from the cannula 200 are improved. In addition, it is easy to suction a plurality of objects into the cannula 200 in a single suction operation and to discharge a plurality of objects outside the cannula 200 in a single discharge operation.

The cannula 200 is integrally molded entirely of, for example, a rigid resin. The rigid resin is not particularly limited, and examples thereof include polypropylene, polycarbonate, and polymethylpentene.

In addition, the total capacity of the cannula 200 is not particularly limited, but is, for example, preferably 10 $\mu$L or more and 300 $\mu$L or less, more preferably 50 $\mu$L or more and 250 $\mu$L or less, and still more preferably 100 $\mu$L or more and 200 $\mu$L or less.

The suction capacity of the cannula 200 mounted on the suction device is not particularly limited, but is, for example, preferably 1 $\mu$L or more and 100 $\mu$L or less, more preferably 10 $\mu$L or more and 80 $\mu$L or less, and still more preferably 20 $\mu$L or more and 60 $\mu$L or less.

It is noted that the expression "suction capacity" as used herein means a capacity with which an object (the cell sheet 410 or the storage solution 420 (or the administration solution)) can be suctioned into the cannula 200 without coming into contact with a suction device (for example, a syringe main body described later).

In the case of the present embodiment, the cannula 200 is mounted on the suction device in a case where the distal end part of the suction device is fitted into the lumen of the mounting part 210 of the cannula 200.

In the present embodiment, as an example, the suction device on which the cannula 200 is mounted is a syringe (not shown) including a syringe main body formed in a cylindrical shape and a plunger that is slidable within the syringe main body in the axial direction. The syringe is not particularly limited, and a well-known syringe can be used.

In a state in which the cannula 200 is mounted on the distal end part of the syringe, the lumen of the cannula 200 and the lumen of the syringe main body are in airtight communication with each other.

Then, by advancing and retracting the plunger with respect to the syringe main body, it is possible to perform a suction operation of suctioning the object into the cannula 200 and a discharge operation of discharging the object in the cannula 200 from the distal end opening 222.

More specifically, a retraction operation of retracting the plunger toward the proximal end side with respect to the syringe main body is performed. As a result, the air in the cannula 200 is suctioned into the syringe main body. Then, the object is suctioned into the lumen of the cannula 200 through the distal end opening 222 of the cannula 200.

On the other hand, the air in the syringe main body is introduced into the cannula 200 by an advancing operation of advancing the plunger to the distal end side with respect to the syringe main body. Then, the object in the cannula 200 is discharged from the distal end opening 222 of the cannula 200.

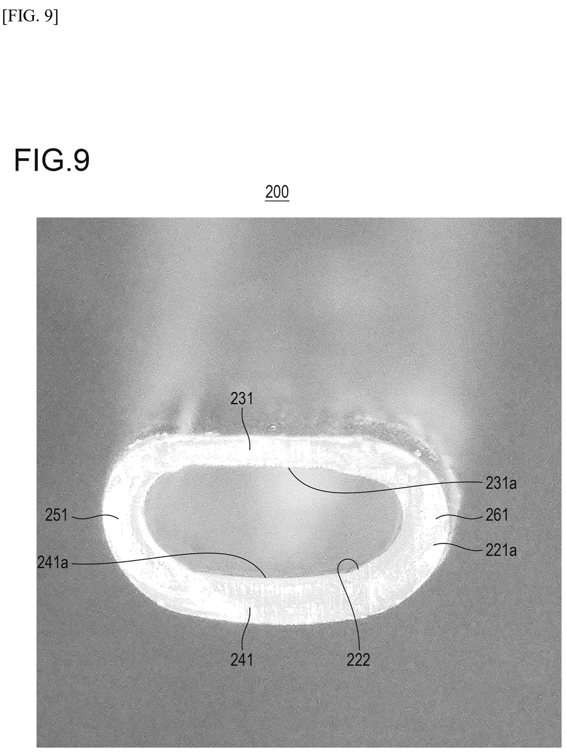

Here, an enlarged photograph of the distal end opening 222 of the actually obtained cannula 200 is shown in FIG. 9.

As shown in FIG. 9, the distal end opening 222 has a flattened shape in which a dimension of the bent part 221 in a bending direction is smaller than a dimension in a direction orthogonal to the bending direction.

In addition, the distal end 221a of the bent part 221 includes a first portion 231 located on the bending direction side and a second portion 241 located on a side opposite to the bending direction side. In addition, the wall thickness of the first portion 231 is smaller than the wall thickness of the second portion 241.

As a result, as described above, since the portion (the pointed end 233 of the first portion 231) that first enters the biological tissue in a case where the cannula 200 is inserted into the affected part of the living body has a thin-walled structure, resistance during insertion can be suppressed, which can achieve lower invasiveness.

On the other hand, since the second portion 241 has a greater wall thickness than the first portion 231, in a case where the object in the cannula 200 is discharged from the distal end opening 222, a separation distance between the distal end opening 222 and the biological tissue around the distal end opening 222 can be ensured. Therefore, immediately after the object is discharged from the distal end opening 222, frictional resistance between the object and the biological tissue around the distal end opening 222 can be suppressed, whereby the object can be easily pushed out to a desired position (the delivery property is improved).

As described above, according to the present embodiment, it is possible to realize the cannula 200 having good suction property, good discharge property, furthermore, good insertion property, and good supply property of the object.

Hereinafter, an example of a method of recovering and transplanting a cell sheet (hereinafter, simply referred to as a cell sheet 410) in which induced pluripotent stem cells cultured in regenerative medicine are formed in a sheet shape using a syringe on which the cannula 200 according to the present embodiment is mounted will be described.

First, in a case of recovering the cell sheet 410 in the cell recovery container 300, the cell sheet 410 in the storage solution 420 (or the administration solution) is suctioned from the cell recovery container 300 into the cannula 200 using a syringe.

More specifically, a retraction operation of retracting the plunger toward the proximal end side with respect to the syringe main body is performed. As a result, the cell sheet 410 is suctioned into the lumen of the cannula 200 through the distal end opening 222 of the cannula 200. In this case, the storage solution 420 (or the administration solution) is also suctioned into the cannula 200 together with the cell sheet 410.

Subsequently, the distal end part (bent part 221) of the cannula 200 is inserted into the affected part of the living body.

Here, as described above, the first portion 231 of the bent part 221, which is the distal end part of the cannula 200, has the pointed end 233 that protrudes furthest in the protruding direction of the first portion, and the wall thickness of the first portion 231 is smaller than the wall thickness of the second portion 241.

As a result, since the portion (the pointed end 233 of the first portion 231) that first enters the biological tissue in a case where the cannula 200 is inserted into the affected part of the living body has a thin-walled structure, resistance during insertion can be suppressed, which can achieve lower invasiveness.

In addition, since the resistance during the insertion can be suppressed, the cannula 200 can be smoothly inserted into the affected part of the living body.

Next, the cell sheet 410 in the cannula 200 is discharged to the affected part of the living body.

More specifically, the advancing operation of advancing the plunger to the distal end side with respect to the syringe main body is performed. As a result, the cell sheet 410 in the cannula 200 is discharged from the distal end opening 222 of the cannula 200 to the affected part of the living body.

Here, as described above, the first portion 231 of the bent part 221, which is the distal end part of the cannula 200, has the pointed end 233 that protrudes furthest in the protruding direction of the first portion, and the wall thickness of the first portion 231 is smaller than the wall thickness of the second portion 241.

As a result, in a case where the cell sheet 410 in the cannula 200 is discharged from the distal end opening 222, a separation distance between the distal end opening 222 and the biological tissue around the distal end opening 222 can be ensured. Therefore, immediately after the cell sheet 410 is discharged from the distal end opening 222, frictional resistance between the cell sheet 410 and the biological tissue around the distal end opening 222 can be suppressed, whereby the cell sheet 410 can be easily pushed out to a desired position (the delivery property is improved).

In this manner, the cultured cell sheet 410 can be recovered using the suction device (syringe) on which the cannula 200 is mounted, and the recovered cell sheet 410 can be transplanted to the affected part of the living body.

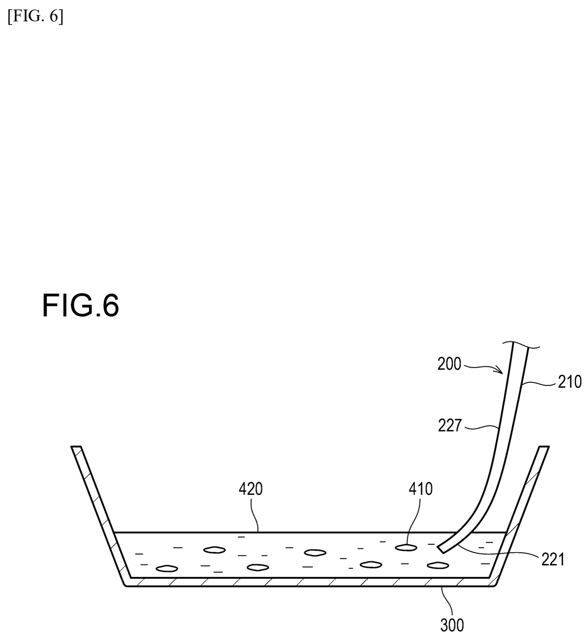

Next, an example of a method of transplanting all of a plurality of (for example, 4 or 8) cell sheets 410 (more specifically, retinal sheets) into the subretinal space of the eyeball 500 by a single suction and discharge operation using a suction device on which the cannula 200 according to the present embodiment is mounted will be described with reference to FIGS. 6 to 8B. In FIGS. 7, 8A, and 8B, a state in which the bent part 221 (distal end part) of the cannula 200 is inserted into the eyeball 500 is schematically shown.

First, a general vitrectomy including posterior vitreous detachment is performed using a cataract and vitreous surgical apparatus (for example, a CONSTELLATION Vision System manufactured by Alcon AG, approval number: 22200BZX00923000).

Next, after an injection kit (for example, MedOne Injection Kit, notification number: 27B1X00046MO3275) is connected to the cataract and vitreous surgical apparatus, the injection kit is filled with an oxyglutathione ophthalmic irrigation and washing solution, and a subretinal administration needle (for example, MedOne Cannula, certification number: 226AFBZI00075000) is connected to the injection kit. Then, using the liquid injection function of the cataract and vitreous surgical apparatus, the oxyglutathione ophthalmic irrigation and washing solution filled in the injection kit is injected into the subretinal space through the subretinal administration needle to produce focal retinal detachment. Then, a part of the detached retina 520 is incised with vitreous scissors or the like to form a transplantation incision 560 (see FIG. 7).

As shown in FIG. 6, in a case of recovering the cell sheet 410 in the cell recovery container 300, the retraction operation (suction operation) of retracting the plunger to the proximal end side with respect to the syringe main body is performed as described above. As a result, all of the plurality of cell sheets 410 are suctioned together with the storage solution 420 (or the administration solution) into the lumen of the cannula 200 through the distal end opening 222 of the cannula 200. The thickness of the cell sheet 410 is, as an example, about 0.5 mm. It is noted that in FIGS. 6, 7, and 8B, the shape of the cell sheet 410 is schematically shown.

However, in this case, all of the desired number of cell sheets 410 may not be necessarily suctioned into the cannula 200 by a single suction operation (retraction operation). In a case where a desired number of cell sheets 410 cannot be suctioned into the cannula 200 in a single suction operation, only the storage solution 420 is discharged from the cannula 200 such that the suctioned cell sheet 410 is not discharged, thereby ensuring the capacity in the cannula 200 for the discharged storage solution 420. As a result, it is possible to further suction a plurality of cell sheets 410 while holding the suctioned cell sheet 410 in the cannula 200. Then, by repeating such an operation, a desired number of cell sheets 410 can be suctioned into the cannula 200.

Next, as shown in FIGS. 7 and 8A, the distal end part (bent part 221) of the cannula 200 is inserted into the eyeball 500. More specifically, first, an incision (hereinafter, a scleral incision 570) having an appropriate width is separately formed in the sclera 530 with a scalpel or the like, and the distal end part (bent part 221) of the cannula 200 is inserted into the eyeball 500 through the scleral incision 570.

Here, as described above, the first portion 231 of the bent part 221, which is the distal end part of the cannula 200, has the pointed end 233 that protrudes furthest in the protruding direction of the first portion, and the wall thickness of the first portion 231 is smaller than the wall thickness of the second portion 241.

As a result, since the resistance during the insertion can be suppressed, the distal end part of the cannula 200 can be smoothly inserted into the eyeball 500 through the scleral incision 570.

Next, as shown in FIGS. 7 and 8B, the first portion 231 of the cannula 200 is inserted between the sensory retina and the retinal pigment epithelium (RPE) layer (may be also simply referred to as "subretinal space") via the vitreous cavity 550 through the above-mentioned transplantation incision 560. In this case, as described above, since the portion (the pointed end 233 of the first portion 231) that enters the biological tissue in the cannula 200 has a thin-walled structure, the first portion 231 of the cannula 200 and thus the distal end part can be smoothly inserted into the subretinal space.

Then, after inserting the distal end part (bent part 221) of the cannula 200 into the subretinal space from the transplantation incision 560, an advancing operation (discharge operation) of advancing the plunger to the distal end side with respect to the above-described syringe main body is performed. As a result, the cell sheet 410 in the cannula 200 is discharged from the distal end opening 222 of the cannula 200 to a space between the sensory retina and the retinal pigment epithelium (RPE) layer (subretinal space).

In this case, with the cannula 200 according to the present embodiment as described above, it is possible to suppress the frictional resistance between the cell sheet 410 and the biological tissue around the distal end opening 222 immediately after the cell sheet 410 is discharged from the distal end opening 222. Therefore, all of the suctioned cell sheets 410 can be smoothly discharged by a single discharge operation.

Due to the above-described operation, all of the plurality of cell sheets 410 (for example, 4 or 8 cell sheets) can be transplanted into subretinal space of the eyeball 500 by a single suction and discharge operation.

It is noted that in the present invention, the number of cell sheets 410 to be suctioned and discharged by a single suction and discharge operation is not particularly limited, and can be appropriately set depending on the application of the cell sheet 410 to be transplanted or the part of the living body.

The dimensions of each portion of the cell sheet 410 according to the present embodiment are not particularly limited as long as the cell sheet 410 can be suctioned into and discharged from the distal end opening 222 and can be supplied to a desired biological tissue, and can be appropriately set according to the use application of the cell sheet 410 and the like. In a case of the cell sheet 410 having soft physical property, the size (major axis or minor axis) of the distal end opening 222 may exceed the size by about two times. Examples of the cell sheet 410 include ES cells, iPS cells, cell sheets (for example, a cell sheet in the form of a substantially spherical or substantially ellipsoidal mass, or a substantially planar cell sheet, an epithelial sheet, an epithelial tissue sheet, an epithelial tissue sheet having a multilayer structure, a tubular tissue, a cord-like tissue, a strip-like tissue, a tissue fragment, a three-dimensional tissue (organoid), and a fragment of a three-dimensional tissue) produced using these cells, and tissues excised from a living body (for example, a fetus or a fetal tissue). Examples of the cell sheet that can be produced by inducing differentiation of ES cells or iPS cells include ectodermal-derived tissues, more specifically, cell sheets of neural system tissues or neural precursor cells, and even more specifically, tissues containing cells of specific brain or neural regions (tissues including neural cells such as forebrain, midbrain, cerebrum, spinal cord, and retinal tissues). In addition, examples of another cell sheet that can be produced by inducing differentiation of ES cells or iPS cells include tissues derived from endoderm and mesoderm, and more specifically, digestive organ tissues (stomach, intestine, liver, pancreas, and the like), respiratory organ tissues, urinary tract tissues, skeletal tissues, muscular tissues, cardiovascular tissues (heart, blood vessels, and the like), pancreatic tissue, intestinal tissues, and tissues including cells of urogenital organs (kidneys and the like). As the size of such a cell sheet, for example, all of the major axis, the minor axis, and the thickness are 0.05 mm or more and 10.0 mm or less, preferably 0.1 mm or more and 5.0 mm or less, more preferably 0.2 mm or more and 3.0 mm or less, and still more preferably 0.3 mm or more and 2.0 mm or less. In addition, in a case where the cell sheet is a retinal sheet, as the size thereof, for example, preferably, a minor axis is 400 μm or more and 800 μm or less, a major axis is about 1200 μm, and a thickness is 300 μm or more and 600 μm or less.

<Modification Example>

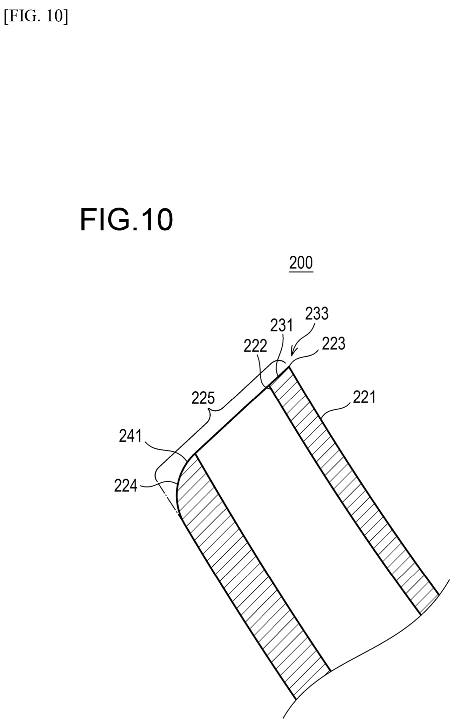

Next, a modification example of the embodiment will be described with reference to FIG. 10. It is noted that FIG. 10 shows the bent part 221 in a cross section including an axis AX1 of the bent part 221.

The bent part 221 of the cannula 200 according to the present modification example is different from the cannula 200 in the above-described embodiment in that a corner 224 on a side opposite to the bending direction side has a rounded R-chamfered shape.

With such a configuration, since the corner 224 on the side opposite to the bending direction side, which is a portion that is likely to come into contact with the biological tissue (for example, the retina 520) in a case where the object is discharged from the distal end opening 222, has the R-chamfered shape, it is possible to realize a configuration with further lower invasiveness in the cannula 200.

The present invention is not limited to the above-described embodiments, and various modifications, improvements, and the like are also included as long as the object of the present invention is achieved.

The present embodiment encompasses the following technical ideas.

(1) A cannula including:

a distal end part that is a bent part bent with respect to a proximal end side part,
in which a distal end of the bent part includes a first portion that is located on a bending direction side of the bent part, and a second portion that is located on a side opposite to the bending direction side,
the first portion protrudes in an extension direction of the bent part more than the second portion, and has a pointed end that protrudes furthest in a protruding direction of the first portion, and
the first portion and the second portion are opposed to each other in parallel.

(2) A cannula including:

a distal end part that is a bent part bent with respect to a proximal end side part,
in which a distal end opening has a flattened shape in which a dimension in a bending direction of the bent part is smaller than a dimension in a direction orthogonal to the bending direction,
a distal end of the bent part includes a first portion that is located on the bending direction side, and a second portion that is located on a side opposite to the bending direction side,
the first portion protrudes in an extension direction of the bent part more than the second portion, and has a pointed end that protrudes furthest in a protruding direction of the first portion, and
the cannula has at least any one of

an opening area of the distal end opening of 0.20 mm$^2$ or more, or
a ratio of the opening area of the distal end opening to an outer shape area at a distal end of the cannula, the ratio being 35% or more.

(3) A cannula including:

a distal end part that is a bent part bent with respect to a proximal end side part,
in which a distal end of the bent part includes a first portion that is located on a bending direction side of the bent part, and a second portion that is located on a side opposite to the bending direction side,
the first portion protrudes in an extension direction of the bent part more than the second portion, and has a pointed end that protrudes furthest in a protruding direction of the first portion,
a wall thickness of the first portion is smaller than a wall thickness of the second portion, and
the first portion and the second portion are opposed to each other in parallel.

(3-1) A cannula including:

a distal end part that is a bent part bent with respect to a proximal end side part,
in which a distal end opening has a flattened shape in which a dimension in a bending direction of the bent part is smaller than a dimension in a direction orthogonal to the bending direction,

a distal end of the bent part includes a first portion that is located on the bending direction side, and a second portion that is located on a side opposite to the bending direction side,

the first portion protrudes in an extension direction of the bent part more than the second portion, and has a pointed end that protrudes furthest in a protruding direction of the first portion,

a wall thickness of the first portion is smaller than a wall thickness of the second portion, and

the first portion and the second portion are opposed to each other in parallel.

(4) A cannula including:

a distal end part that is a bent part bent with respect to a proximal end side part,

in which a distal end opening has a flattened shape in which a dimension in a bending direction of the bent part is smaller than a dimension in a direction orthogonal to the bending direction,

a distal end of the bent part includes a first portion that is located on the bending direction side, and a second portion that is located on a side opposite to the bending direction side,

the first portion protrudes in an extension direction of the bent part more than the second portion, and has a pointed end that protrudes furthest in a protruding direction of the first portion, and

a wall thickness of the first portion is smaller than a wall thickness of the second portion.

(5) The cannula according to any one of (1) to (4),

in which the distal end of the bent part includes an inclined part inclined with respect to an axis of the bent part, and the inclined part extends, in a cross section including the axis of the bent part and an axis of the proximal end side part, from a corner on the bending direction side at the distal end of the bent part toward a side opposite to the bending direction side.

(6) The cannula according to (5),

in which in a cross section including the axis of the bent part and the axis of the proximal end side part, an angle at the corner on the bending direction side at the distal end of the bent part is acute.

(7) The cannula according to any one of (1) to (6),

in which the distal end of the bent part includes a third portion that connects one end of the first portion and one end of the second portion in a circumferential direction, and a fourth portion that connects the other end of the first portion and the other end of the second portion in the circumferential direction, and

a wall thickness of the third portion and a wall thickness of the fourth portion are larger than a wall thickness of the first portion.

(8) The cannula according to any one of (1) to (7),

in which a portion from the proximal end side part to the distal end of the bent part is bent in an arc.

(9) The cannula according to any one of (1) to (8),

in which a lumen cross-sectional shape of a proximal end part in the proximal end side part is a true circular shape, and

a lumen cross-sectional shape of a portion from an intermediate portion of the proximal end side part to a distal end opening is a flattened shape.

(10) The cannula according to any one of (1) to (9),

in which on a luminal surface of the cannula, the number of fissures having a width of 10 $\mu$m or more is less than 10 per 0.045 mm$^2$.

(11) A cannula including:

a distal end part that is a bent part bent with respect to a proximal end side part,

in which a distal end opening has a flattened shape in which a dimension in a bending direction of the bent part is smaller than a dimension in a direction orthogonal to the bending direction,

a distal end of the bent part includes a first portion that is located on the bending direction side, and a second portion that is located on a side opposite to the bending direction side,

the first portion protrudes in an extension direction of the bent part more than the second portion, and has a pointed end that protrudes furthest in a protruding direction of the first portion, and
the distal end opening has a major axis and a minor axis, and a length of the minor axis is 0.30 mm or more.

(12) A cannula including:

a distal end part that is a bent part bent with respect to a proximal end side part,
in which a distal end of the bent part includes a first portion that is located on a bending direction side of the bent part, and a second portion that is located on a side opposite to the bending direction side,
the first portion protrudes in an extension direction of the bent part more than the second portion, and has a pointed end that protrudes furthest in a protruding direction of the first portion,
the first portion and the second portion are opposed to each other in parallel, and
a distal end opening has a major axis and a minor axis, and a length of the minor axis is 0.30 mm or more.

(13) The cannula according to any one of (1) to (12),
in which an opening area of the distal end opening of 0.20 mm$^2$ or more.
(14) The cannula according to any one of (1) to (13),
in which a ratio of the opening area of the distal end opening to an outer shape area at a distal end of the cannula, the ratio being 35% or more.

Examples

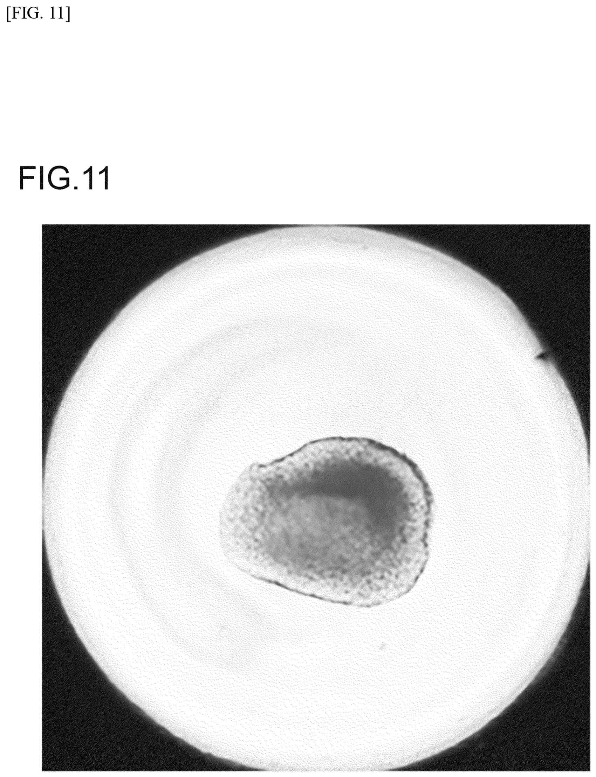

Hereinafter, Examples 1 to 6 and Comparative Example 1 will be described with reference to FIGS. 11 to 14 and Tables 1 to 5. In Examples 1 to 4, the first cannula 200a was used as the cannula 200, in Example 5, the first cannula 200a and the second cannula 200b were used as the cannula 200, and in Example 6, the first cannula 200a, the second cannula 200b, and the third cannula 200c were used as the cannula 200, and each test was performed. The first cannula 200a, the second cannula 200b, and the third cannula 200c are common in that the distal end opening 222 has a flattened shape in which a dimension of the bent part 221 in a bending direction is smaller than a dimension of the bent part 221 in a direction orthogonal to the bending direction, but are different from each other in each parameter shown in Table 1 below.

More specifically, the first cannula 200a is the cannula 200 described in the above-described embodiment, and includes all of the first feature to the fourth feature. The second cannula 200b has the first feature and the second feature, but does not have the third feature and the fourth feature. The third cannula 200c has the second feature, but does not have the first feature, the third feature, and the fourth feature.

In Table 1, the "degree of displacement of the first portion 231" is a ratio of a range in which the inner edge 231a of the first portion 231 is present in the minor-axis direction to the minor axis of the distal end opening 222 in a range of 2/3 or more of the major axis of the distal end opening 222, as described above, and similarly, the "degree of displacement of the second portion 241" is a ratio of a range in which the inner edge 241a of the second portion 241 is present in the minor-axis direction to the minor axis of the distal end opening 222 in a range of 2/3 or more of the major axis of the distal end opening 222, as described above. In addition, the "minimum wall thickness ratio" is a ratio of the minimum value T1 of the wall thickness of the first portion 231 to the minimum value T2 of the wall thickness of the second portion 241 as described above. The "minor axis" is a minor axis of the distal end opening 222, and the "opening area" is an opening area of the distal end opening 222. The "opening area ratio" is a ratio of the opening area to the outer shape area (outer circle area) at the distal end of the cannula 200 as described above.

Each test and the result thereof described below are an example of evaluating the performance of the cannula 200 in the present invention, and do not limit the performance, the use method, and the application of the cannula 200.

[Table 1]

| | First cannula 200a | Second cannula 200b | Third cannula 200c |
|---|---|---|---|
| Degree of displacement of first portion 231 | 5.2% | 12.5% | 23.2% |
| Degree of displacement of second portion 241 | 10.4% | 12.5% | 20.4% |
| Minimum wall thickness ratio | 68.2% | 121.7% | 187.8% |
| Minor axis | 0.368 mm | 0.305 mm | 0.436 mm |
| Opening area | 0.271 mm$^2$ | 0.213 mm$^2$ | 0.266 mm$^2$ |
| Opening area ratio | 45.3% | 36.2% | 41.1% |

<Example 1>

First, Example 1 will be described. In Example 1, the suction and discharge performance of the cannula using the retinal sheet (cell sheet) was evaluated (in vitro evaluation) using the first cannula 200a.

More specifically, it was evaluated whether all of the plurality of retinal sheets aligned on the cell recovery container 300 could be suctioned in a single suction operation using the suction device on which the first cannula 200a was mounted, and whether all of the plurality of suctioned retinal sheets could be discharged into the cell recovery container 300 in a single discharge operation.

More specifically, the test was performed according to the procedures described in the following procedures (1) to (5). It is noted that in the procedure (4) and the procedure (5), the time required for the suction and discharge operation of the retinal sheet was also evaluated.

Procedure (1): The storage solution in the storage container containing the retinal sheet is replaced with the administration solution.

Procedure (2): Procedure (1) is performed for all of retinal sheets, and all of the retinal sheets are transferred to the cell recovery container 300.

Procedure (3): All of the plurality of retinal sheets are aligned on the cell recovery container 300 such that the suction operation is easy.

Procedure (4): The plurality of retinal sheets arranged in the cell recovery container 300 are suctioned using the suction device on which the first cannula 200a is mounted. In this case, it is confirmed whether all of the plurality of retinal sheets can be suctioned in a single suction operation.

Procedure (5): The suctioned retinal sheet is discharged again into the cell recovery container. In this case, it is confirmed whether all of the plurality of retinal sheets can be discharged in a single discharge operation.

It is noted that in the above-described test, the case where the number of retinal sheets to be suctioned and discharged as Example 1-1 was 4 and the case where the number of retinal sheets to be suctioned and discharged as Example 1-2 was 8 were each performed 3 times.

The results of each test are shown in Table 2. In all of the tests, all of the retinal sheets could be suctioned and discharged in a short time in a single suction and discharge operation. That is, it was found that, by using the suction device on which the first cannula 200a is mounted, all of a plurality of retinal sheets can be suctioned and discharged in a single suction and discharge operation.

[Table 2]

| Example | Number of retina sheets | Test No. | Whether all retinal sheets were suctioned and discharged in single suction and discharge operation | Time required for suction and discharge operation (procedures (4) and (5)) |
|---------|-------------------------|----------|---------|---------|
| 1-1 | 4 | 1st | Yes | 9 seconds |
|     |   | 2nd | Yes | 10 seconds |
|     |   | 3rd | Yes | 4 seconds |
| 1-2 | 8 | 1st | Yes | 29 seconds |
|     |   | 2nd | Yes | 18 seconds |
|     |   | 3rd | Yes | 13 seconds |

<Example 2>

Next, Example 2 will be described. In Example 2, the influence of the suction and discharge operation using the suction device on which the cannula 200 is mounted, on the quality of the retinal sheet was evaluated by using the first cannula 200a.

More specifically, as Example 2, the morphological observation, the number of viable cells, and the cell viability of the retinal sheet on which the series of operations (the procedures (1) to (5) of Example 1) including the suction and discharge operation using the suction device on which the first cannula 200a is mounted were performed were evaluated (N = 8).

In addition, as Comparative Example 1, the morphological observation, the number of viable cells, and the cell viability of the retinal sheet on which only the washing operation (the procedure (1) of Example 1) was performed were evaluated in the same manner as in Example 2 (N = 4).

In the morphological observation, the morphology of the retinal sheet was observed using an inverted microscope (manufactured by Olympus Corporation). Here, one captured image of the retinal sheet of Example 2 is shown in FIG. 11 as a representative example.

As shown in FIG. 11 and Table 3, in all of the 8 retinal sheets of Example 2, the morphology of the retinal sheet was well maintained without being collapsed or partially lost, and there was no difference in morphology as compared with Comparative Example 1. Furthermore, no morphological difference was found in the epithelial structure of the retinal tissue contained in the retinal sheet of Example 2 as compared to Comparative Example 1. From these results, it was found that there was no effect on the morphology of the retinal sheet by the suction and discharge operation using the suction device on which the first cannula 200a was mounted.

[Table 3]

|  | Morphology |
|---|---|
| Comparative Example 1 (N = 4) | Good |
| Example 2 (N = 8) | Good |

In the evaluation of the number of viable cells and the cell viability, an enzyme solution heated to 37°C was added to centrifuge tubes, and one retinal sheet was placed in each centrifuge tube. After heating on a water bath set to 37°C, the mixture was gently pipetted in a centrifuge tube. The dispersion of the cells in the centrifuge tube was visually confirmed, and the number of viable cells and the cell viability were measured using a cell counter (NC-200, manufactured by ChemoMetec A/S).

The measurement results of the cell viability and the number of viable cells of Comparative Example 1 and the measurement results of the cell viability and the number of viable cells of Example 2 are shown in Table 4. Since there was no difference between the cell viability and the number of viable cells in Example 2 and the cell viability and the number of viable cells in Comparative Example 1, it was found that the suction and discharge operation using the suction device to which the first cannula 200a was mounted had no effect on the number of viable cells and the cell viability.

[Table 4]

|  | Cell viability (%) | Number of viable cells (cells/mL) |
|---|---|---|
| Comparative Example 1 (N = 4) | 99 | $2.4 \times 10^5$ |
| Example 2 (N = 8) | 99 | $2.3 \times 10^5$ |

From the above results, it was confirmed that even in a case where the suction and discharge operation of the retinal sheet was performed using the suction device on which the cannula 200 was mounted, the quality of the retinal sheet was not affected.

<Example 3>

Next, Example 3 will be described. In Example 3, the transplantation evaluation (ex vivo evaluation) of the retinal sheet to the subretinal space of the excised porcine eye was performed using the first cannula 200a.

More specifically, the retinal sheet was washed and suctioned into the first cannula 200a in the same manner as in the procedures (1) to (4) of Example 1 using eight retinal sheets, and the retinal sheet was administered into the subretinal space of the porcine eye in the same manner as the method of transplanting all of the plurality of retinal sheets described in the above-described embodiment into the subretinal space of the eyeball 500 by a single suction and discharge operation. Then, it was evaluated whether or not all of the retinal sheets could be administered into subretinal space in a single suction and discharge operation. The test was performed twice as Example 3-1 and Example 3-2.

The results of each test are shown in Table 5 below. As shown in Table 5, with the suction device on which the first cannula 200a is mounted, all of a plurality of retinal sheets can be transplanted to the subretinal space of the porcine eye in a single suction and discharge operation.

[Table 5]

| Example | Evaluation item |  |
|---|---|---|
|  | Whether all retina sheets were suctioned in single suction and discharge operation and transplanted into subretinal space | |
| Example 3-1 (1st test) | Yes | |

(continued)

| Example | Evaluation item |
|---|---|
| | Whether all retina sheets were suctioned in single suction and discharge operation and transplanted into subretinal space |
| Example 3-2 (2nd test) | Yes |

From such a result, it was confirmed that using the suction device on which the first cannula 200a was mounted, all of the eight retinal sheets could be suctioned in a single suction and discharge operation and could be administered into subretinal space.

<Example 4>

Next, Example 4 will be described. In Example 4, the transplantation evaluation (in vivo evaluation) of the retinal sheet into subretinal space of the monkey was performed using the first cannula 200a.

More specifically, the retinal sheet was washed and the retinal sheet was suctioned into the first cannula 200a in the same manner as in the above-described procedures (1) to (4) of Example 1 using eight retinal sheets, and the retinal sheet was administered into the subretinal space of the crab-eating macaque in the same manner as the method of transplanting all of the plurality of retinal sheets described in the above-described embodiment into the subretinal space of the eyeball 500 by a single suction and discharge operation. Then, it was evaluated whether or not all of the retinal sheets could be suctioned and the suctioned retinal sheets could be administered into subretinal space in a single suction and discharge operation, with a surgical microscope (N = 1).

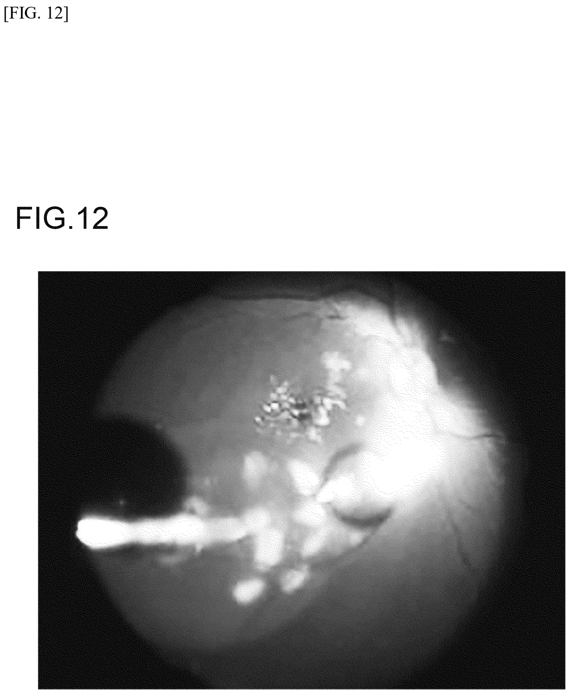

FIG. 12 shows an image of the subretinal space of the monkey into which the retinal sheet has been administered, captured with a surgical microscope. As shown in FIG. 12, using the suction device on which the first cannula 200a was mounted, all of the eight retinal sheets could be suctioned and all of the suctioned eight retinal sheets could be actually transplanted to subretinal space of the monkey.

From such a result, it has been verified that by using the suction device on which the first cannula 200a is mounted, the retinal sheet can be actually transplanted into the subretinal space of the large animal having the eyeball structure with a size and a structure similar to that of the human.

<Example 5>

Next, Example 5 will be described. In Example 5, the effect of the smoothness of the luminal surface of the cannula 200 on the suction and discharge of the cell sheet was examined.

More specifically, first, as the cannula 200, a first cannula 200a and a second cannula 200b were prepared.

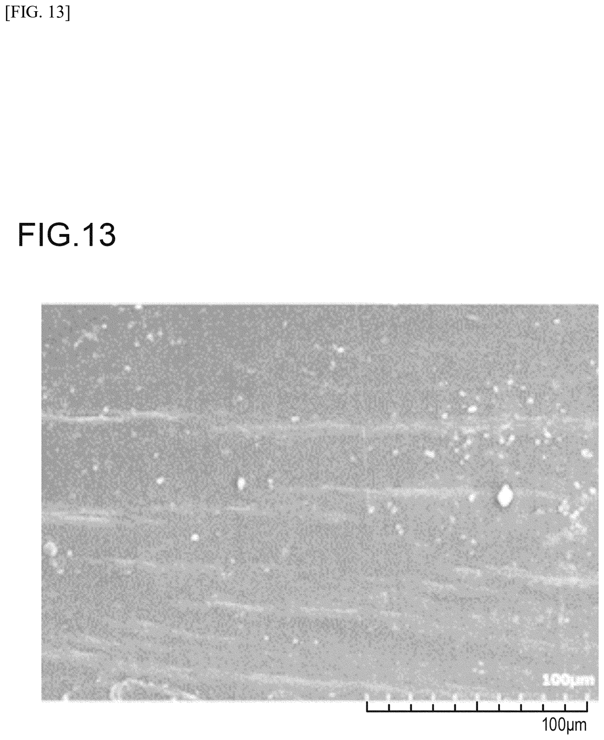

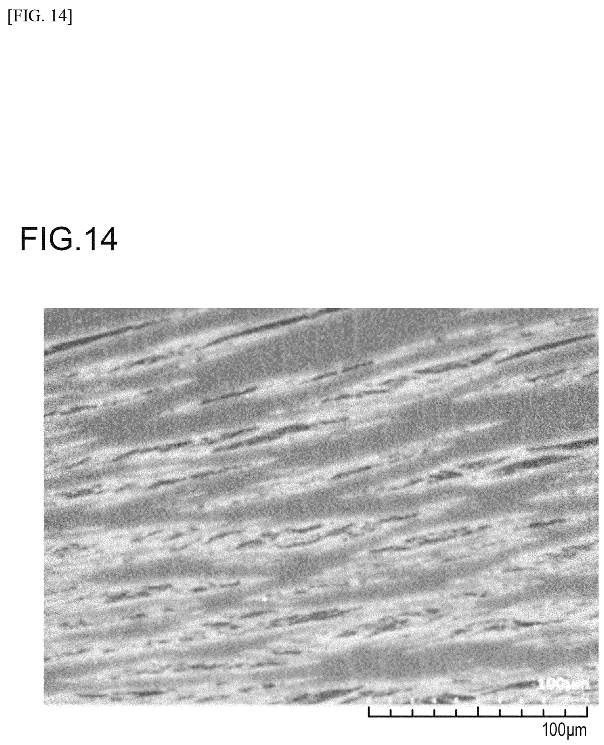

Next, the luminal surface of the first cannula 200a and the luminal surface of the second cannula 200b were observed using a scanning electron microscope (S-3400N, manufactured by Hitachi High-Tech Corporation). FIG. 13 shows a captured image of the luminal surface of the first cannula 200a, and FIG. 14 shows a captured image of the luminal surface of the second cannula 200b.

As shown in FIG. 13, in the first cannula 200a, there were less than 10 fissures with a width of 10 $\mu$m or more per 0.045 mm$^2$, and only a few thin and small fissures.

On the other hand, as shown in FIG. 14, in the second cannula 200b, 10 or more large fissures with a width of 10 $\mu$m or more were confirmed per 0.045 mm$^2$.

Then, using each of the first cannula 200a and the second cannula 200b, eight retinal sheets were transplanted into the subretinal space of the eyeball 500 by the same method as the method of transplanting all of the plurality of retinal sheets described in the above-described embodiment into the subretinal space of the monkey by a single suction and discharge operation, and the suction and discharge performance of the cannula was evaluated. As a result, it was observed that the first cannula 200a tends to easily suction and discharge the retinal sheet as compared with the second cannula 200b.

From these results, it was suggested that the smoothness of the luminal surface of the cannula 200 is also one of the important elements for improving suction property and discharge property. More specifically, from the viewpoint of improving suction property and discharge property, it is preferable that there are few large fissures having a width of 10 $\mu$m or more on the luminal surface of the cannula 200, and it is more preferable that there are no large fissures having a width of 10 $\mu$m or more on the luminal surface of the cannula 200.

<Example 6>

Next, Example 6 will be described. In Example 6, the influence of the parameter characterizing the shape of the distal end opening portion of the cannula 200 on the suction property of the retinal sheet and the discharge property to the porcine eye was evaluated.

More specifically, the retinal sheet was washed and suctioned into the cannula 200 in the same manner as in the procedures (1) to (4) of Example 1 using eight retinal sheets, and the retinal sheet was administered into the subretinal space of the porcine eye in the same manner as the method of transplanting all of the plurality of retinal sheets described in the above-described embodiment into the subretinal space of the eyeball 500 by a single suction and discharge operation. Then, it was evaluated whether or not all of the retinal sheets could be administered into subretinal space in a single suction and discharge operation.

The test was divided into two stages of a suction operation into the cannula 200 and a discharge operation into the porcine eye. As Example 6-1, the suction operability of the retinal sheet of the first cannula 200a, the second cannula 200b, and the third cannula 200c was evaluated. Thereafter, as Example 6-2, the discharge property of the retinal sheet to the porcine eye was evaluated using the first cannula 200a (N = 3).

As a result of Example 6-1, the suction property of the retinal sheet was such that all of the first cannula 200a, the second cannula 200b, and the third cannula 200c were able to suction the 8 retinal sheets without any problem. Compared to the second cannula 200b, a tendency was observed in which both the first cannula 200a and the third cannula 200c were easier to suction. In addition, a tendency was observed in which the first cannula 200a was slightly easier to suction than the third cannula 200c.

As a result of Example 6-2, the discharge property to the subretinal space of the porcine eye using the first cannula 200a did not have any problem, and 8 retinal sheets, which were the required amount, could be administered at once.

From such results, it was suggested that the opening width, the opening area, and the opening area ratio of the distal end opening 222 were also one of the important elements for the suction property and the discharge property of the retinal sheet. More specifically, it is preferable that the distal end opening 222 has the minor axis of 0.35 mm or more, the opening area of 0.25 mm$^2$ or more, or the opening area ratio of 40% or more, and it is more preferable that additionally, the first portion 231 and the second portion 241 are opposed to each other in parallel as in the first cannula 200a and the wall thickness of the first portion 231 is smaller than the wall thickness of the second portion 241.

This application claims priority based on Japanese Patent Application No. 2023-113148 filed on July 10, 2023, the entire disclosure of which is incorporated herein by reference.

REFERENCE SIGNS LIST

> 200 Cannula
> 200a First cannula
> 200b Second cannula
> 200c Third cannula
> 210 Mounting part
> 211a Small-diameter part
> 211b Large-diameter part
> 220 Conduit part
> 221 Bent part (distal end part)
> 221a Distal end
> 222 Distal end opening
> 223 Corner on bending direction side
> 224 Corner on side opposite to bending direction side
> 225 Inclined part
> 227 Proximal end side part
> 228 Proximal end part
> 231 First portion
> 231a Inner edge
> 233 Pointed end
> 241 Second portion
> 241a Inner edge
> 251 Third portion

# EP 4 744 631 A1

261 Fourth portion
300 Cell recovery container
410 Cell sheet
420 Storage solution
500 Eyeball
520 Retina
530 Sclera
550 Vitreous cavity
560 Transplantation incision
570 Scleral incision
601, 602 Imaginary line
611, 612, 613 Boundary line
AX Axis of cannula
AX1 Axis of bent part
AX11 Axis of most distal end part of bent part
AX2 Axis of proximal end side part
P1 Bending start point on in-course side
P2 Bending end point on in-course side
P3 Point where curvature on in-course side is maximum
P4 Bending start point on out-course side
P5 Bending end point on out-course side
P6 Point where curvature on out-course side is maximum

## Claims

1.  A cannula comprising:

    a distal end part that is a bent part bent with respect to a proximal end side part,
    wherein a distal end of the bent part includes a first portion that is located on a bending direction side of the bent part, and a second portion that is located on a side opposite to the bending direction side,
    the first portion protrudes in an extension direction of the bent part more than the second portion, and has a pointed end that protrudes furthest in a protruding direction of the first portion, and
    the first portion and the second portion are opposed to each other in parallel.

2.  A cannula comprising:

    a distal end part that is a bent part bent with respect to a proximal end side part,
    wherein a distal end opening has a flattened shape in which a dimension in a bending direction of the bent part is smaller than a dimension in a direction orthogonal to the bending direction,
    a distal end of the bent part includes a first portion that is located on the bending direction side, and a second portion that is located on a side opposite to the bending direction side,
    the first portion protrudes in an extension direction of the bent part more than the second portion, and has a pointed end that protrudes furthest in a protruding direction of the first portion, and
    the cannula has at least any one of

    an opening area of the distal end opening of 0.20 mm$^2$ or more, or
    a ratio of the opening area of the distal end opening to an outer shape area at a distal end of the cannula, the ratio being 35% or more.

3.  A cannula comprising:

    a distal end part that is a bent part bent with respect to a proximal end side part,
    wherein a distal end of the bent part includes a first portion that is located on a bending direction side of the bent part, and a second portion that is located on a side opposite to the bending direction side,
    the first portion protrudes in an extension direction of the bent part more than the second portion, and has a pointed end that protrudes furthest in a protruding direction of the first portion,
    a wall thickness of the first portion is smaller than a wall thickness of the second portion, and

24

the first portion and the second portion are opposed to each other in parallel.

4. A cannula comprising:

a distal end part that is a bent part bent with respect to a proximal end side part,
wherein a distal end opening has a flattened shape in which a dimension in a bending direction of the bent part is smaller than a dimension in a direction orthogonal to the bending direction,
a distal end of the bent part includes a first portion that is located on the bending direction side, and a second portion that is located on a side opposite to the bending direction side,
the first portion protrudes in an extension direction of the bent part more than the second portion, and has a pointed end that protrudes furthest in a protruding direction of the first portion, and
a wall thickness of the first portion is smaller than a wall thickness of the second portion.

5. The cannula according to any one of claims 1 to 4,

wherein the distal end of the bent part includes an inclined part inclined with respect to an axis of the bent part, and the inclined part extends, in a cross section including the axis of the bent part and an axis of the proximal end side part, from a corner on the bending direction side at the distal end of the bent part toward a side opposite to the bending direction side.

6. The cannula according to claim 5,
wherein in the cross section including the axis of the bent part and the axis of the proximal end side part, an angle at the corner on the bending direction side at the distal end of the bent part is acute.

7. The cannula according to any one of claims 1 to 4,

wherein the distal end of the bent part includes a third portion that connects one end of the first portion and one end of the second portion in a circumferential direction, and a fourth portion that connects the other end of the first portion and the other end of the second portion in the circumferential direction, and
a wall thickness of the third portion and a wall thickness of the fourth portion are larger than a wall thickness of the first portion.

8. The cannula according to any one of claims 1 to 4,
wherein a portion from the proximal end side part to the distal end of the bent part is bent in an arc.

9. The cannula according to any one of claims 1 to 4,

wherein a lumen cross-sectional shape of a proximal end part in the proximal end side part is a true circular shape, and
a lumen cross-sectional shape of a portion from an intermediate portion of the proximal end side part to a distal end opening is a flattened shape.

10. The cannula according to any one of claims 1 to 4,
wherein on a luminal surface of the cannula, the number of fissures having a width of 10 $\mu$m or more is less than 10 per 0.045 mm$^2$.

11. A cannula comprising:

a distal end part that is a bent part bent with respect to a proximal end side part,
wherein a distal end opening has a flattened shape in which a dimension in a bending direction of the bent part is smaller than a dimension in a direction orthogonal to the bending direction,
a distal end of the bent part includes a first portion that is located on the bending direction side, and a second portion that is located on a side opposite to the bending direction side,
the first portion protrudes in an extension direction of the bent part more than the second portion, and has a pointed end that protrudes furthest in a protruding direction of the first portion, and
the distal end opening has a major axis and a minor axis, and a length of the minor axis is 0.30 mm or more.

12. A cannula comprising:

a distal end part that is a bent part bent with respect to a proximal end side part,

wherein a distal end of the bent part includes a first portion that is located on a bending direction side of the bent part, and a second portion that is located on a side opposite to the bending direction side,

the first portion protrudes in an extension direction of the bent part more than the second portion, and has a pointed end that protrudes furthest in a protruding direction of the first portion,

the first portion and the second portion are opposed to each other in parallel, and

a distal end opening has a major axis and a minor axis, and a length of the minor axis is 0.30 mm or more.

[FIG. 1]

FIG.1

[FIG. 2]

# FIG.2A

# FIG.2B

[FIG. 3]

# FIG.3

[FIG. 4]

FIG.4A

FIG.4B

[FIG. 5]

FIG.5A

200

227

AX2(AX)

FIG.5B

200

228

AX2(AX)

[FIG. 6]

FIG.6

[FIG. 7]

FIG.7

[FIG. 8]

# FIG.8A

# FIG.8B

[FIG. 9]

# FIG.9

[FIG. 10]

FIG.10

[FIG. 11]

FIG.11

[FIG. 12]

FIG.12

[FIG. 13]

# FIG.13

[FIG. 14]

FIG.14

100μm

# EP 4 744 631 A1

## INTERNATIONAL SEARCH REPORT

| International application No. |
|---|
| **PCT/JP2024/024963** |

| A. | CLASSIFICATION OF SUBJECT MATTER |
|---|---|

*A61F 9/007*(2006.01)i
FI:    A61F9/007 130E

According to International Patent Classification (IPC) or to both national classification and IPC

| B. | FIELDS SEARCHED |
|---|---|

Minimum documentation searched (classification system followed by classification symbols)

A61F9/007

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 3055946 B2 (PHOTOGENESIS, INCORPORATED) 26 June 2000 (2000-06-26) column 16, lines 19-38, fig. 17 | 1, 5-6 |

| ☐ Further documents are listed in the continuation of Box C. | ☑ See patent family annex. |
|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 September 2024** | **01 October 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**EP 4 744 631 A1**

## INTERNATIONAL SEARCH REPORT

International application No.

**PCT/JP2024/024963**

| Box No. III | Observations where unity of invention is lacking (Continuation of item 3 of first sheet) |

This International Searching Authority found multiple inventions in this international application, as follows:

The number of inventions set forth in claims 1-12 is 22. For the following reasons, the present international application is considered not to comply with the requirement of unity of invention.

Document 1: JP 3055946 B2 (PHOTOGENESIS, INCORPORATED) 26 June 2000 (2000-06-26) column 16, lines 19-38, fig. 17 & US 5817075 A column 9, lines 14-39, fig. 17

(Invention 1) Claim 1 and claims 5-6 referring to claim 1

Document 1 discloses, for claim 1, a cannula (a supply tube (a feeding cannula) 210) in which a distal end portion (a tubular distal end portion 212) has a bent portion (a curved portion 220) that is bent relative to a proximal end part (a main part of the supply pipe (the feeding cannula) 210),

a distal end (an edge 218) of the bent portion includes a first part located on a bending direction side of the bent portion and a second part located on the opposite side to the bending direction (fig. 17),

the first part protrudes more than the second part in an extension direction of the bent portion and has a protruding tip end (a distal end 218) that protrudes most in the protruding direction (fig. 17), and

the first part and the second part face each other in parallel (it is obvious that the first part and the second part described in document 1 face each other at least partially in parallel).

Document 1 discloses, for claim 5, features in which the distal end of the bent portion includes an inclined portion (an edge 218) inclined relative to the axis core of the bent portion, and

on a cross-section including the axis of the bent portion and the axis of the proximal end part, the inclined portion extends from a corner of the distal end of the bent portion on the bending direction side toward the opposite side to the bending direction (fig. 17).

Document 1 discloses, for claim 6, a feature in which, on a cross-section including the axis core of the bent portion and the axis core of the proximal end part, the corner at the front end of the bent portion in the bending direction has an acute angle (fig. 17).

Accordingly, claims 1 and 5-6 lack novelty in light of document 1 and thus do not have a special technical feature.

Therefore, claims 1 and 5-6 are classified as invention 1.

(Invention 2) Claim 7 referring to claim 1

Claim 7 is dependent on claim 1 classified as invention 1, but the specific problem to be solved by the invention, as understood from the technical feature added to claim 1, of achieving good shape retention at a distal end opening (paragraph [0036], etc.), has little relevance to the feature, which is the problem to be solved by claim 1, of: reducing resistance during insertion and making the procedure less invasive because a part (the distal end of the first part) entering the living tissue first has a thin structure when inserting the cannula into the affected region of the body (paragraphs [0023], [0048]-[0049], etc.); and ensuring that the opening width and opening area of the distal end opening are sufficient (paragraphs [0018]-[0019]). Therefore, claim 7 referring to claim 1 is not considered to be inventively related to claim 1. Also, claim 7 referring to claim 1 is not substantially identical to or similarly closely related to any of the claims classified as invention 1.

Therefore, claim 7 referring to claim 1 cannot be classified as invention 1.

Also, claim 7 referring to claim 1 has the special technical feature in which "the distal end of the bent portion includes: a third part for connecting one end of the first part to one end of the second part in the circumferential direction; and a fourth part for connecting the other end of the first part to the other end of the second part in the circumferential direction, wherein the thickness of the third part and the thickness of the fourth part are each greater than the thickness of the first part" and is thus classified as invention 2.

(Invention 3) Claim 8 referring to claim 1

Claim 8 is dependent on claim 1 classified as invention 1, but the specific problem to be solved by the invention, as understood from the technical feature added to claim 1, of achieving smooth flow of an object from the proximal end part to the bent portion compared to the case in which a bent portion is bent in the form of a broken line (a straight line) with respect to the proximal end part (paragraph [0033], etc.), has little relevance to the feature, which is the problem to be solved by claim 1, of: reducing resistance during insertion and making the procedure less invasive because a part (the distal end of the first part) entering the living tissue first has a thin structure when inserting the cannula into the affected region of the body (paragraphs [0023], [0048]-[0049], etc.); and ensuring that the opening width and opening area of the distal end opening are sufficient (paragraphs [0018]-[0019]). Therefore, claim 8 referring to claim 1 is not considered to be inventively related to claim 1.

Form PCT/ISA/210 (continuation of first sheet) (July 2022)

42

**INTERNATIONAL SEARCH REPORT**

International application No.

**PCT/JP2024/024963**

| Box No. III | Observations where unity of invention is lacking (Continuation of item 3 of first sheet) |
|---|---|

Also, claim 8 referring to claim 1 is not substantially identical to or similarly closely related to any of the claims classified as invention 1 or 2.

Therefore, claim 8 referring to claim 1 cannot be classified as either invention 1 or 2.

Also, claim 8 referring to claim 1 has the special technical feature in which "a section from the proximal end part to the distal end of the bent portion is bent in an arc shape" and is thus classified as invention 3.

(Invention 4) Claim 9 referring to claim 1

Claim 9 is dependent on claim 1 classified as invention 1, but the specific problem to be solved by the invention, as understood from the technical feature added to claim 1, of orienting an object (changing the direction of a discharged material) to match the flat shape and then passing the object through the bent portion (paragraph [0034], etc.) when an aspect ratio (a horizontal to vertical ratio) of the object is different, has little relevance to the feature, which is the problem to be solved by claim 1, of: reducing resistance during insertion and making the procedure less invasive because a part (the distal end of the first part) entering the living tissue first has a thin structure when inserting the cannula into the affected region of the body (paragraphs [0023], [0048]-[0049], etc.); and ensuring that the opening width and opening area of the distal end opening are sufficient (paragraphs [0018]-[0019]). Therefore, claim 9 referring to claim 1 is not considered to be inventively related to claim 1.

Also, claim 9 referring to claim 1 is not substantially identical to or similarly closely related to any of the claims classified as inventions 1-3.

Therefore, claim 9 referring to claim 1 cannot be classified as any of inventions 1-3.

Also, claim 9 referring to claim 1 has the special technical feature in which "the luminal cross-section at a proximal end portion of the proximal end part has a perfect circle shape, and the luminal cross-section from a middle portion of the proximal end part to the distal end opening is a flat shape" and is thus classified as invention 4.

(Invention 5) Claim 10 referring to claim 1

Claim 10 is dependent on claim 1 classified as invention 1, but the specific problem to be solved by the invention, as understood from the technical feature added to claim 1, of suppressing frictional resistance between an object and the luminal surface of a cannula (paragraph [0043], etc.), has little relevance to the feature, which is the problem to be solved by claim 1, of: reducing resistance during insertion and making the procedure less invasive because a part (the distal end of the first part) entering the living tissue first has a thin structure when inserting the cannula into the affected region of the body (paragraphs [0023], [0048]-[0049], etc.); and ensuring that the opening width and opening area of the distal end opening are sufficient (paragraphs [0018]-[0019]). Therefore, claim 10 referring to claim 1 is not considered to be inventively related to claim 1.

Also, claim 10 referring to claim 1 is not substantially identical to or similarly closely related to any of the claims classified as inventions 1-4.

Therefore, claim 10 referring to claim 1 cannot be classified as any of inventions 1-4.

Also, claim 10 referring to claim 1 has the special technical feature in which "the luminal cross-section at a proximal end portion of the proximal end part has a perfect circle shape, and the luminal cross-section from a middle portion of the proximal end part to the distal end opening is a flat shape" and is thus classified as invention 5.

(Invention 6) Claim 2 and claims 5-6 referring to claim 2

Claim 2 and claims 5-6 referring to claim 2 share, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 2 and claims 5-6 referring to claim 2 are not dependent on claim 1. Furthermore, claim 2 and claims 5-6 referring to claim 2 are not substantially identical to or similarly closely related to any of the claims classified as inventions 1-5.

Therefore, claim 2 and claims 5-6 referring to claim 2 cannot be classified as any of inventions 1-5.

Also, claim 2 and claims 5-6 referring to claim 2 have the special technical features in which "the distal end opening has a flat shape in which the dimension in the bending direction of the bending portion is less than the dimension in the direction perpendicular to the bending direction" and "at least one of the following conditions is satisfied: the opening region of the distal end opening is 0.20 mm2 or more; or the ratio of the opening region of the distal end opening to the outer area at the distal end of the cannula is 35% or more" and are thus classified as invention 6.

Form PCT/ISA/210 (continuation of first sheet) (July 2022)

## INTERNATIONAL SEARCH REPORT

International application No.

**PCT/JP2024/024963**

| Box No. III | Observations where unity of invention is lacking (Continuation of item 3 of first sheet) |

(Invention 7) Claim 7 referring to claim 2

Claim 7 referring to claim 2 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 7 referring to claim 2 is not dependent on claim 1. Also, claim 7 referring to claim 2 is not substantially identical to or similarly closely related to any of the claims classified as inventions 1-6.

Therefore, claim 7 referring to claim 2 cannot be classified as any of inventions 1-6.

Also, claim 7 referring to claim 2 has the special technical features in which "the distal end opening has a flat shape in which the dimension in the bending direction of the bending portion is less than the dimension in the direction perpendicular to the bending direction," "at least one of the following conditions is satisfied: the opening region of the distal end opening is 0.20 mm2 or more; or the ratio of the opening region of the distal end opening to the outer area at the distal end of the cannula is 35% or more," or "the distal end of the bent portion includes: a third part for connecting one end of the first part to one end of the second part in the circumferential direction; and a fourth part for connecting the other end of the first part to the other end of the second part in the circumferential direction, wherein the thickness of the third part and the thickness of the fourth part are each greater than the thickness of the first part" and is thus classified as invention 7.

(Invention 8) Claim 8 referring to claim 2

Claim 8 referring to claim 2 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 8 referring to claim 2 is not dependent on claim 1. Also, claim 8 referring to claim 2 is not substantially identical to or similarly closely related to any of the claims classified as inventions 1-7.

Therefore, claim 8 referring to claim 2 cannot be classified as any of inventions 1-7.

Also, claim 8 referring to claim 2 has the special technical features in which "the distal end opening has a flat shape in which the dimension in the bending direction of the bending portion is less than the dimension in the direction perpendicular to the bending direction," "at least one of the following conditions is satisfied: the opening region of the distal end opening is 0.20 mm2 or more; or the ratio of the opening region of the distal end opening to the outer area at the distal end of the cannula is 35% or more," or "a section from the proximal end part to the distal end of the bent portion is bent in an arc shape" and is thus classified as invention 8.

(Invention 9) Claim 9 referring to claim 2

Claim 9 referring to claim 2 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 9 referring to claim 2 is not dependent on claim 1. Also, claim 9 referring to claim 2 is not substantially identical to or similarly closely related to any of the claims classified as inventions 1-8.

Therefore, claim 9 referring to claim 2 cannot be classified as any of inventions 1-8.

Also, claim 9 referring to claim 2 has the special technical features in which "the distal end opening has a flat shape in which the dimension in the bending direction of the bending portion is less than the dimension in the direction perpendicular to the bending direction," "at least one of the following conditions is satisfied: the opening region of the distal end opening is 0.20 mm2 or more; or the ratio of the opening region of the distal end opening

## INTERNATIONAL SEARCH REPORT

International application No.

PCT/JP2024/024963

**Box No. III    Observations where unity of invention is lacking (Continuation of item 3 of first sheet)**

to the outer area at the distal end of the cannula is 35% or more," or "the luminal cross-section at a proximal end portion of the proximal end part has a perfect circle shape, and the luminal cross-section from a middle portion of the proximal end part to the distal end opening is a flat shape" and is thus classified as invention 9.

(Invention 10) Claim 10 referring to claim 2

Claim 10 referring to claim 2 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 10 referring to claim 2 is not dependent on claim 1. Also, claim 10 referring to claim 2 is not substantially identical to or similarly closely related to any of the claims classified as inventions 1-9.

Therefore, claim 10 referring to claim 2 cannot be classified as any of inventions 1-9.

Also, claim 10 referring to claim 2 has the special technical features in which "the distal end opening has a flat shape in which the dimension in the bending direction of the bending portion is less than the dimension in the direction perpendicular to the bending direction," "at least one of the following conditions is satisfied: the opening region of the distal end opening is 0.20 mm2 or more; or the ratio of the opening region of the distal end opening to the outer area at the distal end of the cannula is 35% or more," or "the cannula has less than 10 cracks with a diameter of 10 μm or more per 0.045 mm2 on the inner surface thereof" and is thus classified as invention 10.

(Invention 11) Claim 3 and claims 5-6 referring to claim 3

Claim 3 and claims 5-6 referring to claim 3 share, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 3 and claims 5-6 referring to claim 3 are not dependent on claim 1. Furthermore, claim 3 and claims 5-6 referring to claim 3 are not substantially identical to or similarly closely related to any of the claims classified as inventions 1-10.

Therefore, claim 3 and claims 5-6 referring to claim 3 cannot be classified as any of inventions 1-10.

Also, claim 3 and claims 5-6 referring to claim 3 have the special technical feature in which "the thickness of the first part is less than the thickness of the second part" and are thus classified as invention 11.

(Invention 12) Claim 7 referring to claim 3

Claim 7 referring to claim 3 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 7 referring to claim 3 is not dependent on claim 1. Furthermore, claim 7 referring to claim 3 is not substantially identical to or similarly closely related to any of the claims classified as inventions 1-11.

Therefore, claim 7 referring to claim 3 cannot be classified as any of inventions 1-11.

Also, claim 7 referring to claim 3 has the special technical features in which "the thickness of the first part is less than the thickness of the second part" and "the distal end of the bent portion includes: a third part for connecting one end of the first part to one end of the second part in the circumferential direction; and a fourth part for connecting the other end of the first part to the other end of the second part in the circumferential direction, wherein the thickness

## INTERNATIONAL SEARCH REPORT

International application No.

**PCT/JP2024/024963**

| Box No. III | Observations where unity of invention is lacking (Continuation of item 3 of first sheet) |
|---|---|

of the third part and the thickness of the fourth part are each greater than the thickness of the first part" and is thus classified as invention 12.

(Invention 13) Claim 8 referring to claim 3

Claim 8 referring to claim 3 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 8 referring to claim 3 is not dependent on claim 1. Furthermore, claim 8 referring to claim 3 is not substantially identical to or similarly closely related to any of the claims classified as inventions 1-12.

Therefore, claim 8 referring to claim 3 cannot be classified as any of inventions 1-12.

Also, claim 8 referring to claim 3 has the special technical features in which "the thickness of the first part is less than the thickness of the second part," "the distal end opening has a flat shape in which the dimension in the bending direction of the bending portion is less than the dimension in the direction perpendicular to the bending direction," and "a section from the proximal end part to the distal end of the bent portion is bent in an arc shape" and is thus classified as invention 13.

(Invention 14) Claim 9 referring to claim 3

Claim 9 referring to claim 3 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 9 referring to claim 3 is not dependent on claim 1. Furthermore, claim 9 referring to claim 3 is not substantially identical to or similarly closely related to any of the claims classified as inventions 1-13.

Therefore, claim 9 referring to claim 3 cannot be classified as any of inventions 1-13.

Also, claim 9 referring to claim 3 has the special technical features in which "the thickness of the first part is less than the thickness of the second part" and "the luminal cross-section at a proximal end portion of the proximal end part has a perfect circle shape, and the luminal cross-section from a middle portion of the proximal end part to the distal end opening is a flat shape" and is thus classified as invention 14.

(Invention 15) Claim 10 referring to claim 3

Claim 10 referring to claim 3 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 10 referring to claim 3 is not dependent on claim 1. Furthermore, claim 10 referring to claim 3 is not substantially identical to or similarly closely related to any of the claims classified as inventions 1-14.

Therefore, claim 10 referring to claim 3 cannot be classified as any of inventions 1-14.

Also, claim 10 referring to claim 3 has the special technical features in which "the thickness of the first part is less than the thickness of the second part" and "the cannula has less than 10 cracks with a diameter of 10 µm or more per 0.045 mm2 on the inner surface thereof" and is thus classified as invention 15.

(Invention 16) Claim 4 and claims 5-6 referring to claim 4

Form PCT/ISA/210 (continuation of first sheet) (July 2022)

**INTERNATIONAL SEARCH REPORT**

International application No.

**PCT/JP2024/024963**

**Box No. III     Observations where unity of invention is lacking (Continuation of item 3 of first sheet)**

Claim 4 and claims 5-6 referring to claim 4 share, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 4 and claims 5-6 referring to claim 4 are not dependent on claim 1. Furthermore, claim 4 and claims 5-6 referring to claim 4 are not substantially identical to or similarly closely related to any of the claims classified as inventions 1-15.

Therefore, claim 4 and claims 5-6 referring to claim 4 cannot be classified as any of inventions 1-15.

Also, claim 4 and claims 5-6 referring to claim 4 have the special technical features in which "the distal end opening has a flat shape in which the dimension in the bending direction of the bending portion is less than the dimension in the direction perpendicular to the bending direction" and "the thickness of the first part is less than the thickness of the second part" and are thus classified as invention 16.

(Invention 17) Claim 7 referring to claim 4

Claim 7 referring to claim 4 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 7 referring to claim 4 is not dependent on claim 1. Furthermore, claim 7 referring to claim 4 is not substantially identical to or similarly closely related to any of the claims classified as inventions 1-16.

Therefore, claim 7 referring to claim 4 cannot be classified as any of inventions 1-16.

Also, claim 7 referring to claim 4 has the special technical features in which "the distal end opening has a flat shape in which the dimension in the bending direction of the bending portion is less than the dimension in the direction perpendicular to the bending direction," "the thickness of the first part is less than the thickness of the second part," and "the distal end of the bent portion includes: a third part for connecting one end of the first part to one end of the second part in the circumferential direction; and a fourth part for connecting the other end of the first part to the other end of the second part in the circumferential direction, wherein the thickness of the third part and the thickness of the fourth part are each greater than the thickness of the first part" and is thus classified as invention 17.

(Invention 18) Claim 8 referring to claim 4

Claim 8 referring to claim 4 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 8 referring to claim 4 is not dependent on claim 1. Also, claim 8 referring to claim 4 is not substantially identical to or similarly closely related to any of the claims classified as inventions 1-17.

Therefore, claim 8 referring to claim 4 cannot be classified as any of inventions 1-17.

Also, claim 8 referring to claim 4 has the special technical features in which "the distal end opening has a flat shape in which the dimension in the bending direction of the bending portion is less than the dimension in the direction perpendicular to the bending direction," "the distal end opening has a flat shape in which the dimension in the bending direction of the bending portion is less than the dimension in the direction perpendicular to the bending direction," and "a section from the proximal end part to the distal end of the bent portion is bent in an arc shape" and is thus classified as invention 18.

Form PCT/ISA/210 (continuation of first sheet) (July 2022)

**INTERNATIONAL SEARCH REPORT**

| International application No. |
| --- |
| **PCT/JP2024/024963** |

| **Box No. III** | **Observations where unity of invention is lacking (Continuation of item 3 of first sheet)** |
| --- | --- |

(Invention 19) Claim 9 referring to claim 4

Claim 9 referring to claim 4 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 9 referring to claim 4 is not dependent on claim 1. Furthermore, claim 9 referring to claim 4 is not substantially identical to or similarly closely related to any of the claims classified as inventions 1-18.

Therefore, claim 9 referring to claim 4 cannot be classified as any of inventions 1-18.

Also, claim 9 referring to claim 4 has the special technical features in which "the distal end opening has a flat shape in which the dimension in the bending direction of the bending portion is less than the dimension in the direction perpendicular to the bending direction," "the distal end opening has a flat shape in which the dimension in the bending direction of the bending portion is less than the dimension in the direction perpendicular to the bending direction," and "the luminal cross-section at a proximal end portion of the proximal end part has a perfect circle shape, and the luminal cross-section from a middle portion of the proximal end part to the distal end opening is a flat shape" and is thus classified as invention 19.

(Invention 20) Claim 10 referring to claim 4

Claim 10 referring to claim 4 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 10 referring to claim 4 is not dependent on claim 1. Furthermore, claim 10 referring to claim 4 is not substantially identical to or similarly closely related to any of the claims classified as inventions 1-19.

Therefore, claim 10 referring to claim 4 cannot be classified as any of inventions 1-19.

Also, claim 10 referring to claim 4 has the special technical features in which "the distal end opening has a flat shape in which the dimension in the bending direction of the bending portion is less than the dimension in the direction perpendicular to the bending direction," "the distal end opening has a flat shape in which the dimension in the bending direction of the bending portion is less than the dimension in the direction perpendicular to the bending direction," and "the cannula has less than 10 cracks with a diameter of 10 $\mu$m or more per 0.045 mm2 on the inner surface thereof" and is thus classified as invention 20.

(Invention 21) Claim 11

Claim 11 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction." However, the technical feature does not make a contribution over the prior art in light of the disclosure of document 1, and thus, it cannot be said that the technical feature is a special technical feature. Also, there are no other same or corresponding special technical features between these inventions.

In addition, claim 11 is not dependent on claim 1. Furthermore, claim 11 is not substantially identical to or similarly closely related to any of the claims classified as inventions 1-20.

Therefore, claim 11 cannot be classified as any of inventions 1-20.

Also, claim 11 has the special technical features in which "the distal end opening has a flat shape in which the dimension in the bending direction of the bending portion is less than the dimension in the direction perpendicular to the bending direction" and "the distal end opening has a long diameter and a short diameter, and the length of the short diameter is 0.30 mm or more" and is thus classified as invention 21.

Form PCT/ISA/210 (continuation of first sheet) (July 2022)

## INTERNATIONAL SEARCH REPORT

International application No.

**PCT/JP2024/024963**

| Box No. III | Observations where unity of invention is lacking (Continuation of item 3 of first sheet) |
|---|---|

(Invention 22) Claim 12

Claim 12 shares, with claim 1 classified as invention 1, the common technical feature in which "a distal end portion has a bent portion that is bent relative to a proximal end part, wherein a distal end of the bent portion includes a first part located on a bending direction side of the bent portion and a second part located on the opposite side to the bending direction, and wherein the first part protrudes more than the second part in an extension direction of the bent portion and has a protruding tip end that protrudes most in the protruding direction, and wherein the first part and the second part face each other in parallel." However, the technical feature does not make a contribution over the prior art in light of the disclosure of document 1, and thus, it cannot be said that the technical feature is a special technical feature. Also, there are no other same or corresponding special technical features between these inventions.

In addition, claim 12 is not dependent on claim 1. Furthermore, claim 11 is not substantially identical to or similarly closely related to any of the claims classified as inventions 1-21.

Therefore, claim 12 cannot be classified as any of inventions 1-21.

Also, claim 12 has the special technical feature in which "the distal end opening has a long diameter and a short diameter, and the length of the short diameter is 0.30 mm or more" and is thus classified as invention 22.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **(Invention 1) Claim 1 and claims 5-6 referring to claim 1**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/JP2024/024963**

| Patent document cited in search report | | | Publication date (day/month/year) | Patent family member(s) | | | Publication date (day/month/year) |
|---|---|---|---|---|---|---|---|
| JP | 3055946 | B2 | 26 June 2000 | US | 5817075 | A | |
| | | | | column 9, lines 14-39, fig. 17 | | | |
| | | | | AT | E270082 | T1 | |
| | | | | AT | E163531 | T1 | |
| | | | | AT | E179318 | T1 | |
| | | | | AU | 4844797 | A | |
| | | | | AU | 4856993 | A | |
| | | | | AU | 4858993 | A | |
| | | | | AU | 5354496 | A | |
| | | | | AU | 6271590 | A | |
| | | | | BR | 9307850 | A | |
| | | | | CA | 2065230 | A1 | |
| | | | | CA | 2158442 | A1 | |
| | | | | CA | 2158443 | A1 | |
| | | | | CA | 2189040 | A1 | |
| | | | | DE | 69032100 | T2 | |
| | | | | DE | 69324709 | T2 | |
| | | | | DK | 486589 | T3 | |
| | | | | EP | 486589 | A1 | |
| | | | | EP | 706357 | A1 | |
| | | | | EP | 713377 | A1 | |
| | | | | EP | 764036 | A1 | |
| | | | | ES | 2115594 | T3 | |
| | | | | FI | 954366 | A | |
| | | | | IL | 107497 | A | |
| | | | | IL | 117284 | A | |
| | | | | JP | 5-501969 | A | |
| | | | | JP | 9-501065 | A | |
| | | | | JP | 9-508563 | A | |
| | | | | KR | 10-1996-0700669 | A | |
| | | | | KR | 10-1997-0702737 | A | |
| | | | | MX | 9605185 | A | |
| | | | | NZ | 256280 | A | |
| | | | | SG | 49267 | A1 | |
| | | | | SG | 49754 | A1 | |
| | | | | TW | 332150 | B | |
| | | | | US | 5962027 | A | |
| | | | | US | 6036678 | A | |
| | | | | US | 2002/0055724 | A1 | |
| | | | | US | 2003/0054023 | A1 | |
| | | | | US | 2003/0104618 | A1 | |
| | | | | US | 2006/0039993 | A1 | |
| | | | | US | 6514238 | B1 | |
| | | | | WO | 1991/002499 | A1 | |
| | | | | WO | 1994/021204 | A1 | |
| | | | | WO | 1994/021205 | A1 | |
| | | | | WO | 1996/026759 | A1 | |
| | | | | ZA | 942786 | B | |
| | | | | ZA | 961597 | B | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021126534 A
- US 5941250 A

- JP 2023113148 A

**Non-patent literature cited in the description**

- **M. MANDAI**. Autologous Induced Stem-Cell-Derived Retinal Cells for Macular Degeneration. *The New England Journal of Medicine*, 16 March 2017, vol. 376, 1038-1046